# NEUE EUROPÄISCHE PATENTSCHRIFT

(11) **EP 0 861 334 B2**
(45) Veröffentlichungstag und Bekanntmachung des Hinweises auf die Entscheidung über den Einspruch: **09.04.2008**
(45) Hinweis auf die Patenterteilung: 12.06.2002
(21) Anmeldenummer: 96945992.4
(22) Anmeldetag: 14.11.1996
(51) Int. Cl.: C12Q 1/68

(54) **VERFAHREN ZUR QUANTIFIZIERUNG VON TUMORZELLEN IN EINER KÖRPERFLÜSSIGKEIT UND DAZU GEEIGNETE TESTKITS**
METHOD OF QUANTIFYING TUMOUR CELLS IN A BODY FLUID AND A SUITABLE TEST KIT
PROCEDE POUR QUANTIFIER LE NOMBRE DE CELLULES CANCEREUSES DANS UN VOLUME HUMORAL ET NECESSAIRE D'ESSAI ADEQUAT

(30) Priorität: 16.11.1995 DE 19542795
(43) Veröffentlichungstag der Anmeldung: 02.09.1998
(73) Patentinhaber: Dahm, Michael W., 81677 München (DE)
(72) Erfinder: Dahm, Michael W., 81677 München (DE)
(74) Vertreter: Keller, Günter
(86) Internationale Anmeldenummer: PCT/DE1996/002183
(87) Internationale Veröffentlichungsnummer: WO 1997/018322

(56) Entgegenhaltungen:
- WO-A-96/01835
- GB-A- 2 260 811
- SCIENCE, Bd. 269, - 1.September 1995 Seiten 1236-1241, XP000645335 FENG J. ET AL.,: "The RNA component of human telomerase" in der Anmeldung erwähnt
- SCIENCE, Bd. 269, - 1.September 1995 Seiten 1267-1270, XP000645336 BLASCO M. A. ET AL., : "Functional charaterization and developmental regulation of mouse telomerase RNA"
- Tsai, Biotechniques Nov. 1996, St. 862 - 866
- Genbank Eintrag für pGEM-13Zf(+); Zugangsnummer X65315
- Van Gemen et al., J. Virol. Meth,1993, St. 177 - 188

### SEQUENZPROTOKOLL

(1) ALLGEMEINE INFORMATION:
   ANMELDER:
      (A) NAME: Dr. Dr. Michael Dahm
      (B) STRASSE: Adalbertstr. 38
      (C) ORT: München
      (E) LAND: Deutschland
      (F) POSTLEITZAHL: 80799
   ANMELDETITEL:
      Verfahren zur Quantifizierung von Tumorzellen in einer Körperflüssigkeit..
   ANZAHL DER SEQUENZEN: 21
   COMPUTER-LESBARE FORM:
      (A) DATENTRÄGER: Floppy Disk
      (B) COMPUTER: IBM PC compatible
      (C) BETRIEBSSYSTEM: PC-DOS/MS-DOS
      (D) SOFTWARE: PADAT Sequenzmodul Version 1.0
(2) INFORMATION ZU SEQ ID NO: 1:
   (i) SEQUENZ CHARAKTERISTIKA:
      (A) LÄNGE: 25 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 1:
      GACTCGGCTC ACACATGCAG TTCGC 25
(2) INFORMATION ZU SEQ ID NO: 2:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 2:
      CTGGTCGAGA TCTACCTTGG GAGAAGC 27
(2) INFORMATION ZU SEQ ID NO: 3:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 3:
      CGACTTTGGA GGTGCCTTCA 20
(2) INFORMATION ZU SEQ ID NO: 4:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO:
      4:AAGTCGGATC CACTTAGGTC 20
(2) INFORMATION ZU SEQ ID NO: 5:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 5:
      CGCTCGATTT GGCGACGGGA 20
(2) INFORMATION ZU SEQ ID NO: 6:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 6:
      GAGAGTATAG CGATTGGACG 20
(2) INFORMATION ZU SEQ ID NO: 7:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 7:
      ACCCAGAGGT TCTTTGAGTC 20
(2) INFORMATION ZU SEQ ID NO: 8:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 8:
      TCTGATAGGC AGCCTGCACT 20
(2) INFORMATION ZU SEQ ID NO: 9:
   (i) SEQUENZ CHARAKTERISTIKA:
      (A) LÄNGE: 43 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 9:
      ATAAGAATGC GGCCGCGGGT TGCGGAGGGT GGGCCTGGGA GGG 43
(2) INFORMATION ZU SEQ ID NO: 10:
   (i) SEQUENZ CHARAKTERISTIKA:
      (A) LÄNGE: 26 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 10:
      CCCAAGCTTG TGGGGGTTAT ATCCTA 26
(2) INFORMATION ZU SEQ ID NO: 11:
   (i) SEQUENZ CHARAKTERISTIKA:
      (A) LÄNGE: 44 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 11:
      CGCGGATCCA CTTAGGTCAT CGATCTGCCA ATTTGCAGCA CACT 44
(2) INFORMATION ZU SEQ ID NO: 12:
   (i) SEQUENZ CHARAKTERISTIKA:
      (A) LÄNGE: 40 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 12:
      CGCGGATCCG ACTTGGTACC ATGAATGGGC AGTGAGCCGG 40
(2) INFORMATION ZU SEQ ID NO: 13:
   (i) SEQUENZ CHARAKTERISTIKA:
      (A) LÄNGE: 36 Basenpaare
      (B) ART: Nukleinsäure
      (C) STRANGFORM: Doppel
      (D) TOPOLOGIE: linear
   (ii) ART DES MOLEKÜLS: Genom-DNA
   (xi) SEQUENZBESCHREIBUNG: SEQ ID NO: 13:
      CCATCGATTC CCGTCGCCAA ATCGAGCGGG TACCCC 36
(2) INFORMATION ZU SEQ ID NO: 14:
   (i) SEQUENZ CHARAKTERISTIKA:
      (A) LÄNGE: 36 Basenpaare
      (B) ART: Nukleinsäure
      (C) STRANGFORM: Doppel
      (D) TOPOLOGIE: linear
   (ii) ART DES MOLEKÜLS: Genom-DNA
   (xi) SEQUENZBESCHREIBUNG: SEQ ID NO: 14:
      CCATCGATCG TCCAATCGCT ATACTCTCGG TACCCC 36
(2) INFORMATION ZU SEQ ID NO: 15:
   (i) SEQUENZ CHARAKTERISTIKA:
      (A) LÄNGE: 32 Basenpaare
      (B) ART: Nukleinsäure
      (C) STRANGFORM: Doppel
      (D) TOPOLOGIE: linear
   (ii) ART DES MOLEKÜLS: Genom-DNA
   (xi) SEQUENZBESCHREIBUNG: SEQ ID NO: 15:
      ATCGATGACC TAAGTGGATC CGACTTGGTA CC 32
(2) INFORMATION ZU SEQ ID NO: 16:
   (i) SEQUENZ CHARAKTERISTIKA:
      (A) LÄNGE: 962 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 16:
   GGGTTGCGGA GGGTGGGCCT GGGAGGGGTG GTGGCCATTT TTTGTCTAAC CCTAACTGAG      60
   AAGGGCGTAG GCGCCGTGCT TTTGCTCCCC GCGCGCTGTT TTTCTCGCTG ACTTTCAGCG     120
   GGCGGAAAAG CCTCGGCCTG CCGCCTTCCA CCGTTCATTC TAGAGCAAAC AAAAAATGTC     180
   AGCTGCTGGC CCGTTCGCCT CCCGGGGACC TGCGGCGGGT CGCCTGCCCA GCCCCCGAAC     240
   CCCGCCTGGA GCCGCGGTCG GCCCGGGGCT TCTCCGGAGG CACCCACTGC CACCGCGAAG     300
   AGTTGGGCTC TGTCAGCCGC GGGTCTCTCG GGGGCGAGGG CGAGGTTCAC CGTTTCAGGC     360
   CGCAGGAAGA GGAACGGAGC GAGTCCCGCC GCGGCGCGAT TCCCTGAGCT GTGGGACGTG     420
   CACCCAGGAC TCGGCTCACA CATGCAGTTC GCTTTCCTGT TGGTGGGGGG AACGCCGATC     480
   GTGCGCATCC GTCACCCCTC GCCGGCAGTG GGGGCTTGTG AACCCCCAAA CCTGACTGAC     540
   TGGGCCAGTG TGCTGCAAAT TGGCAGGAGA CGTGAAGGCA CCTCCAAAGT CGGCCAAAAT     600
   GAATGGGCAG TGAGCCGGGG TTGCCTGGAG CCGTTCCTGC GTGGGTTCTC CCGTCTTCCG     660
   CTTTTTGTTG CCTTTTATGG TTGTATTACA ACTTAGTTCC TGCTCTGCAG ATTTTGTTGA     720
   GGTTTTTGCT TCTCCCAAGG TAGATCTCGA CCAGTCCCTC AACGGGGTGT GGGGAGAACA     780
   GTCATTTTTT TTTGAGAGAT CATTTAACAT TTAATGAATA TTTAATTAGA AGATCTAAAT     840
   GAACATTGGA AATTGTGTTC CTTTAATGGT CATCGGTTTA TGCCAGAGGT TAGAAGTTTC     900
   TTTTTTGAAA AATTAGACCT TGGCGATGAC CTTGAGCAGT AGGATATAAC CCCCACAAGC     960
   TT                                                                    962
(2) INFORMATION ZU SEQ ID NO: 17:
   (i) SEQUENZ CHARAKTERISTIKA:
      (A) LÄNGE: 4118 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 17:
   GGGCGAATTG GCGGCCGCGG GTTGCGGAGG GTGGGCCTGG GAGGGGTGGT GGCCATTTTT 60
   TGTCTAACCC TAACTGAGAA GGGCGTAGGC GCCGTGCTTT TGCTCCCCGC GCGCTGTTTT 120
   TCTCGCTGAC TTTCAGCGGG CGGAAAAGCC TCGGCCTGCC GCCTTCCACC GTTCATTCTA 180
   GAGCAAACAA AAAATGTCAG CTGCTGGCCC GTTCGCCTCC CGGGGACCTG CGGCGGGTCG 240
   CCTGCCCAGC CCCCGAACCC CGCCTGGAGC CGCGGTCGGC CCGGGGCTTC TCCGGAGGCA 300
   CCCACTGCCA CCGCGAAGAG TTGGGCTCTG TCAGCCGCGG GTCTCTCGGG GGCGAGGGCG 360
   AGGTTCACCG TTTCAGGCCG CAGGAAGAGG AACGGAGCGA GTCCCGCCGC GGCGCGATTC 420
   CCTGAGCTGT GGGACGTGCA CCCAGGACTC GGCTCACACA TGCAGTTCGC TTTCCTGTTG 480
   GTGGGGGGAA CGCCGATCGT GCGCATCCGT CACCCCTCGC CGGCAGTGGG GGCTTGTGAA 540
   CCCCCAAACC TGACTGACTG GGCCAGTGTG CTGCAAATTG GCAGGAGACG TGAAGGCACC 600
   TCCAAAGTCG GCCAAAATGA ATGGGCAGTG AGCCGGGGTT GCCTGGAGCC GTTCCTGCGT 660
   GGGTTCTCCC GTCTTCCGCT TTTTGTTGCC TTTTATGGTT GTATTACAAC TTAGTTCCTG 720
   CTCTGCAGAT TTTGTTGAGG TTTTTGCTTC TCCCAAGGTA GATCTCGACC AGTCCCTCAA 780
   CGGGGTGTGG GGAGAACAGT CATTTTTTTT TGAGAGATCA TTTAACATTT AATGAATATT 840
   TAATTAGAAG ATCTAAATGA ACATTGGAAA TTGTGTTCCT TTAATGGTCA TCGGTTTATG 900
   CCAGAGGTTA GAAGTTTCTT TTTTGAAAAA TTAGACCTTG GCGATGACCT TGAGCAGTAG 960
   GATATAACCC CCACAAGCTT GAGTATTCTA TAGTGTCACC TAAATAGCTT GGCGTAATCA 1020
   TGGTCATAGC TGTTTCCTGT GTGAAATTGT TATCCGCTCA CAATTCCACA CAACATACGA 1080
   GCCGGAAGCA TAAAGTGTAA AGCCTGGGGT GCCTAATGAG TGAGCTAACT CACATTAATT 1140
   GCGTTGCGCT CACTGCCCGC TTTCCAGTCG GGAAACCTGT CGTGCCAGCT GCATTAATGA 1200
   ATCGGCCAAC GCGCGGGGAG AGGCGGTTTG CGTATTGGGC GCTCTTCCGC TTCCTCGCTC 1260
   ACTGACTCGC TGCGCTCGGT CGTTCGGCTG CGGCGAGCGG TATCAGCTCA CTCAAAGGCG 1320
   GTAATACGGT TATCCACAGA ATCAGGGGAT AACGCAGGAA AGAACATGTG AGCAAAAGGC 1380
   CAGCAAAAGG CCAGGAACCG TAAAAAGGCC GCGTTGCTGG CGTTTTTCCA TAGGCTCCGC 1440
   CCCCCTGACG AGCATCACAA AAATCGACGC TCAAGTCAGA GGTGGCGAAA CCCGACAGGA 1500
   CTATAAAGAT ACCAGGCGTT TCCCCCTGGA AGCTCCCTCG TGCGCTCTCC TGTTCCGACC 1560
   CTGCCGCTTA CCGGATACCT GTCCGCCTTT CTCCCTTCGG GAAGCGTGGC GCTTTCTCAT 1620
   AGCTCACGCT GTAGGTATCT CAGTTCGGTG TAGGTCGTTC GCTCCAAGCT GGGCTGTGTG 1680
   CACGAACCCC CCGTTCAGCC CGACCGCTGC GCCTTATCCG GTAACTATCG TCTTGAGTCC 1740
   AACCCGGTAA GACACGACTT ATCGCCACTG GCAGCAGCCA CTGGTAACAG GATTAGCAGA 1800
   GCGAGGTATG TAGGCGGTGC TACAGAGTTC TTGAAGTGGT GGCCTAACTA CGGCTACACT 1860
   AGAAGGACAG TATTTGGTAT CTGCGCTCTG CTGAAGCCAG TTACCTTCGG AAAAAGAGTT 1920
   GGTAGCTCTT GATCCGGCAA ACAAACCACC GCTGGTAGCG GTGGTTTTTT TGTTTGCAAG 1980
   CAGCAGATTA CGCGCAGAAA AAAAGGATCT CAAGAAGATC CTTTGATCTT TTCTACGGGG 2040
   TCTGACGCTC AGTGGAACGA AAACTCACGT TAAGGGATTT TGGTCATGAG ATTATCAAAA 2100
   AGGATCTTCA CCTAGATCCT TTTAAATTAA AAATGAAGTT TTAAATCAAT CTAAAGTATA 2160
   TATGAGTAAA CTTGGTCTGA CAGTTACCAA TGCTTAATCA GTGAGGCACC TATCTCAGCG 2220
   ATCTGTCTAT TTCGTTCATC CATAGTTGCC TGACTCCCCG TCGTGTAGAT AACTACGATA 2280
   CGGGAGGGCT TACCATCTGG CCCCAGTGCT GCAATGATAC CGCGAGACCC ACGCTCACCG 2340
   GCTCCAGATT TATCAGCAAT AAACCAGCCA GCCGGAAGGG CCGAGCGCAG AAGTGGTCCT 2400
   GCAACTTTAT CCGCCTCCAT CCAGTCTATT AATTGTTGCC GGGAAGCTAG AGTAAGTAGT 2460
   TCGCCAGTTA ATAGTTTGCG CAACGTTGTT GCCATTGCTA CAGGCATCGT GGTGTCACGC 2520
   TCGTCGTTTG GTATGGCTTC ATTCAGCTCC GGTTCCCAAC GATCAAGGCG AGTTACATGA 2580
   TCCCCCATGT TGTGCAAAAA AGCGGTTAGC TCCTTCGGTC CTCCGATCGT TGTCAGAAGT 2640
   AAGTTGGCCG CAGTGTTATC ACTCATGGTT ATGGCAGCAC TGCATAATTC TCTTACTGTC 2700
   ATGCCATCCG TAAGATGCTT TTCTGTGACT GGTGAGTACT CAACCAAGTC ATTCTGAGAA 2760
   TAGTGTATGC GGCGACCGAG TTGCTCTTGC CCGGCGTCAA TACGGGATAA TACCGCGCCA 2820
   CATAGCAGAA CTTTAAAAGT GCTCATCATT GGAAAACGTT CTTCGGGGCG AAAACTCTCA 2880
   AGGATCTTAC CGCTGTTGAG ATCCAGTTCG ATGTAACCCA CTCGTGCACC CAACTGATCT 2940
   TCAGCATCTT TTACTTTCAC CAGCGTTTCT GGGTGAGCAA AAACAGGAAG GCAAAATGCC 3000
   GCAAAAAAGG GAATAAGGGC GACACGGAAA TGTTGAATAC TCATACTCTT CCTTTTTCAA 3060
   TATTATTGAA GCATTTATCA GGGTTATTGT CTCATGAGCG GATACATATT TGAATGTATT 3120
   TAGAAAAATA AACAAATAGG GGTTCCGCGC ACATTTCCCC GAAAAGTGCC ACCTGACGTC 3180
   TAAGAAACCA TTATTATCAT GACATTAACC TATAAAAATA GGCGTATCAC GAGGCCCTTT 3240
   CGTCTCGCGC GTTTCGGTGA TGACGGTGAA AACCTCTGAC ACATGCAGCT CCCGGAGACG 3300
   GTCACAGCTT GTCTGTAAGC GGATGCCGGG AGCAGACAAG CCCGTCAGGG CGCGTCAGCG 3360
   GGTGTTGGCG GGTGTCGGGG CTGGCTTAAC TATGCGGCAT CAGAGCAGAT TGTACTGAGA 3420
   GTGCACCATA TGCGGTGTGA AATACCGCAC AGATGCGTAA GGAGAAAATA CCGCATCAGG    3480
   CGAAATTGTA AACGTTAATA TTTTGTTAAA ATTCGCGTTA AATATTTGTT AAATCAGCTC    3540
   ATTTTTTAAC CAATAGGCCG AAATCGGCAA AATCCCTTAT AAATCAAAAG AATAGACCGA    3600
   GATAGGGTTG AGTGTTGTTC CAGTTTGGAA CAAGAGTCCA CTATTAAAGA ACGTGGACTC    3660
   CAACGTCAAA GGGCGAAAAA CCGTCTATCA GGGCGATGGC CCACTACGTG AACCATCACC    3720
   CAAATCAAGT TTTTTGCGGT CGAGGTGCCG TAAAGCTCTA AATCGGAACC CTAAAGGGAG    3780
   CCCCCGATTT AGAGCTTGAC GGGGAAAGCC GGCGAACGTG GCGAGAAAGG AAGGGAAGAA    3840
   AGCGAAAGGA GCGGGCGCTA GGGCGCTGGC AAGTGTAGCG GTCACGCTGC GCGTAACCAC    3900
   CACACCCGCC GCGCTTAATG CGCCGCTACA GGGCGCGTCC ATTCGCCATT CAGGCTGCGC    3960
   AACTGTTGGG AAGGGCGATC GGTGCGGGCC TCTTCGCTAT TACGCCAGCT GGCGAAAGGG    4020
   GGATGTGCTG CAAGGCGATT AAGTTGGGTA ACGCCAGGGT TTTCCCAGTC ACGACGTTGT    4080
   AAAACGACGG CCAGTGAATT GTAATACGAC TCACTATA                            4118
(2) INFORMATION ZU SEQ ID NO: 18:
   (i) SEQUENZ CHARAKTERISTIKA:
      (A) LÄNGE: 4118 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 18:
   GGGCGAATTG GCGGCCGCGG GTTGCGGAGG GTGGGCCTGG GAGGGGTGGT GGCCATTTTT 60
   TGTCTAACCC TAACTGAGAA GGGCGTAGGC GCCGTGCTTT TGCTCCCCGC GCGCTGTTTT 120
   TCTCGCTGAC TTTCAGCGGG CGGAAAAGCC TCGGCCTGCC GCCTTCCACC GTTCATTCTA 180
   GAGCAAACAA AAAATGTCAG CTGCTGGCCC GTTCGCCTCC CGGGGACCTG CGGCGGGTCG 240
   CCTGCCCAGC CCCCGAACCC CGCCTGGAGC CGCGGTCGGC CCGGGGCTTC TCCGGAGGCA 300
   CCCACTGCCA CCGCGAAGAG TTGGGCTCTG TCAGCCGCGG GTCTCTCGGG GGCGAGGGCG 360
   AGGTTCACCG TTTCAGGCCG CAGGAAGAGG AACGGAGCGA GTCCCGCCGC GGCGCGATTC 420
   CCTGAGCTGT GGGACGTGCA CCCAGGACTC GGCTCACACA TGCAGTTCGC TTTCCTGTTG 480
   GTGGGGGGAA CGCCGATCGT GCGCATCCGT CACCCCTCGC CGGCAGTGGG GGCTTGTGAA 540
   CCCCCAAACC TGACTGACTG GGCCAGTGTG CTGCAAATTG GCAGATCGAT GACCTAAGTG 600
   GATCCGACTT GGTACCATGA ATGGGCAGTG AGCCGGGGTT GCCTGGAGCC GTTCCTGCGT 660
   GGGTTCTCCC GTCTTCCGCT TTTTGTTGCC TTTTATGGTT GTATTACAAC TTAGTTCCTG 720
   CTCTGCAGAT TTTGTTGAGG TTTTTGCTTC TCCCAAGGTA GATCTCGACC AGTCCCTCAA 780
   CGGGGTGTGG GGAGAACAGT CATTTTTTTT TGAGAGATCA TTTAACATTT AATGAATATT 840
   TAATTAGAAG ATCTAAATGA ACATTGGAAA TTGTGTTCCT TTAATGGTCA TCGGTTTATG 900
   CCAGAGGTTA GAAGTTTCTT TTTTGAAAAA TTAGACCTTG GCGATGACCT TGAGCAGTAG 960
   GATATAACCC CCACAAGCTT GAGTATTCTA TAGTGTCACC TAAATAGCTT GGCGTAATCA 1020
   TGGTCATAGC TGTTTCCTGT GTGAAATTGT TATCCGCTCA CAATTCCACA CAACATACGA 1080
   GCCGGAAGCA TAAAGTGTAA AGCCTGGGGT GCCTAATGAG TGAGCTAACT CACATTAATT 1140
   GCGTTGCGCT CACTGCCCGC TTTCCAGTCG GGAAACCTGT CGTGCCAGCT GCATTAATGA 1200
   ATCGGCCAAC GCGCGGGGAG AGGCGGTTTG CGTATTGGGC GCTCTTCCGC TTCCTCGCTC 1260
   ACTGACTCGC TGCGCTCGGT CGTTCGGCTG CGGCGAGCGG TATCAGCTCA CTCAAAGGCG 1320
   GTAATACGGT TATCCACAGA ATCAGGGGAT AACGCAGGAA AGAACATGTG AGCAAAAGGC 1380
   CAGCAAAAGG CCAGGAACCG TAAAAAGGCC GCGTTGCTGG CGTTTTTCCA TAGGCTCCGC 1440
   CCCCCTGACG AGCATCACAA AAATCGACGC TCAAGTCAGA GGTGGCGAAA CCCGACAGGA 1500
   CTATAAAGAT ACCAGGCGTT TCCCCCTGGA AGCTCCCTCG TGCGCTCTCC TGTTCCGACC 1560
   CTGCCGCTTA CCGGATACCT GTCCGCCTTT CTCCCTTCGG GAAGCGTGGC GCTTTCTCAT 1620
   AGCTCACGCT GTAGGTATCT CAGTTCGGTG TAGGTCGTTC GCTCCAAGCT GGGCTGTGTG 1680
   CACGAACCCC CCGTTCAGCC CGACCGCTGC GCCTTATCCG GTAACTATCG TCTTGAGTCC 1740
   AACCCGGTAA GACACGACTT ATCGCCACTG GCAGCAGCCA CTGGTAACAG GATTAGCAGA 1800
   GCGAGGTATG TAGGCGGTGC TACAGAGTTC TTGAAGTGGT GGCCTAACTA CGGCTACACT 1860
   AGAAGGACAG TATTTGGTAT CTGCGCTCTG CTGAAGCCAG TTACCTTCGG AAAAAGAGTT 1920
   GGTAGCTCTT GATCCGGCAA ACAAACCACC GCTGGTAGCG GTGGTTTTTT TGTTTGCAAG 1980
   CAGCAGATTA CGCGCAGAAA AAAAGGATCT CAAGAAGATC CTTTGATCTT TTCTACGGGG 2040
   TCTGACGCTC AGTGGAACGA AAACTCACGT TAAGGGATTT TGGTCATGAG ATTATCAAAA 2100
   AGGATCTTCA CCTAGATCCT TTTAAATTAA AAATGAAGTT TTAAATCAAT CTAAAGTATA 2160
   TATGAGTAAA CTTGGTCTGA CAGTTACCAA TGCTTAATCA GTGAGGCACC TATCTCAGCG 2220
   ATCTGTCTAT TTCGTTCATC CATAGTTGCC TGACTCCCCG TCGTGTAGAT AACTACGATA 2280
   CGGGAGGGCT TACCATCTGG CCCCAGTGCT GCAATGATAC CGCGAGACCC ACGCTCACCG 2340
   GCTCCAGATT TATCAGCAAT AAACCAGCCA GCCGGAAGGG CCGAGCGCAG AAGTGGTCCT 2400
   GCAACTTTAT CCGCCTCCAT CCAGTCTATT AATTGTTGCC GGGAAGCTAG AGTAAGTAGT 2460
   TCGCCAGTTA ATAGTTTGCG CAACGTTGTT GCCATTGCTA CAGGCATCGT GGTGTCACGC 2520
   TCGTCGTTTG GTATGGCTTC ATTCAGCTCC GGTTCCCAAC GATCAAGGCG AGTTACATGA 2580
   TCCCCCATGT TGTGCAAAAA AGCGGTTAGC TCCTTCGGTC CTCCGATCGT TGTCAGAAGT 2640
   AAGTTGGCCG CAGTGTTATC ACTCATGGTT ATGGCAGCAC TGCATAATTC TCTTACTGTC 2700
   ATGCCATCCG TAAGATGCTT TTCTGTGACT GGTGAGTACT CAACCAAGTC ATTCTGAGAA 2760
   TAGTGTATGC GGCGACCGAG TTGCTCTTGC CCGGCGTCAA TACGGGATAA TACCGCGCCA 2820
   CATAGCAGAA CTTTAAAAGT GCTCATCATT GGAAAACGTT CTTCGGGGCG AAAACTCTCA 2880
   AGGATCTTAC CGCTGTTGAG ATCCAGTTCG ATGTAACCCA CTCGTGCACC CAACTGATCT 2940
   TCAGCATCTT TTACTTTCAC CAGCGTTTCT GGGTGAGCAA AAACAGGAAG GCAAAATGCC 3000
   GCAAAAAAGG GAATAAGGGC GACACGGAAA TGTTGAATAC TCATACTCTT CCTTTTTCAA 3060
   TATTATTGAA GCATTTATCA GGGTTATTGT CTCATGAGCG GATACATATT TGAATGTATT 3120
   TAGAAAAATA AACAAATAGG GGTTCCGCGC ACATTTCCCC GAAAAGTGCC ACCTGACGTC 3180
   TAAGAAACCA TTATTATCAT GACATTAACC TATAAAAATA GGCGTATCAC GAGGCCCTTT 3240
   CGTCTCGCGC GTTTCGGTGA TGACGGTGAA AACCTCTGAC ACATGCAGCT CCCGGAGACG 3300
   GTCACAGCTT GTCTGTAAGC GGATGCCGGG AGCAGACAAG CCCGTCAGGG CGCGTCAGCG 3360
   GGTGTTGGCG GGTGTCGGGG CTGGCTTAAC TATGCGGCAT CAGAGCAGAT TGTACTGAGA 3420
   GTGCACCATA TGCGGTGTGA AATACCGCAC AGATGCGTAA GGAGAAAATA CCGCATCAGG      3480
   CGAAATTGTA AACGTTAATA TTTTGTTAAA ATTCGCGTTA AATATTTGTT AAATCAGCTC      3540
   ATTTTTTAAC CAATAGGCCG AAATCGGCAA AATCCCTTAT AAATCAAAAG AATAGACCGA      3600
   GATAGGGTTG AGTGTTGTTC CAGTTTGGAA CAAGAGTCCA CTATTAAAGA ACGTGGACTC      3660
   CAACGTCAAA GGGCGAAAAA CCGTCTATCA GGGCGATGGC CCACTACGTG AACCATCACC      3720
   CAAATCAAGT TTTTTGCGGT CGAGGTGCCG TAAAGCTCTA AATCGGAACC CTAAAGGGAG      3780
   CCCCCGATTT AGAGCTTGAC GGGGAAAGCC GGCGAACGTG GCGAGAAAGG AAGGGAAGAA      3840
   AGCGAAAGGA GCGGGCGCTA GGGCGCTGGC AAGTGTAGCG GTCACGCTGC GCGTAACCAC      3900
   CACACCCGCC GCGCTTAATG CGCCGCTACA GGGCGCGTCC ATTCGCCATT CAGGCTGCGC      3960
   AACTGTTGGG AAGGGCGATC GGTGCGGGCC TCTTCGCTAT TACGCCAGCT GGCGAAAGGG      4020
   GGATGTGCTG CAAGGCGATT AAGTTGGGTA ACGCCAGGGT TTTCCCAGTC ACGACGTTGT      4080
   AAAACGACGG CCAGTGAATT GTAATACGAC TCACTATA                              4118
(2) INFORMATION ZU SEQ ID NO: 19:
   (i) SEQUENZ CHARAKTERISTIKA:
      (A) LÄNGE: 975 Basenpaare
      (B) ART: Nukleinsäure
      (C) STRANGFORM: Einzel
      (D) TOPOLOGIE: linear
   (ii) ART DES MOLEKÜLS: Genom-RNA
   (xi) SEQUENZBESCHREIBUNG: SEQ ID NO: 19:
   GGGCGAAUUG GCGGCCGCGG GUUGCGGAGG GUGGGCCUGG GAGGGGUGGU GGCCAUUUUU 60
   UGUCUAACCC UAACUGAGAA GGGCGUAGGC GCCGUGCUUU UGCUCCCCGC GCGCUGUUUU 120
   UCUCGCUGAC UUUCAGCGGG CGGAAAAGCC UCGGCCUGCC GCCUUCCACC GUUCAUUCUA 180
   GAGCAAACAA AAAAUGUCAG CUGCUGGCCC GUUCGCCUCC CGGGGACCUG CGGCGGGUCG 240
   CCUGCCCAGC CCCCGAACCC CGCCUGGAGC CGCGGUCGGC CCGGGGCUUC UCCGGAGGCA 300
   CCCACUGCCA CCGCGAAGAG UUGGGCUCUG UCAGCCGCGG GUCUCUCGGG GGCGAGGGCG 360
   AGGUUCACCG UUUCAGGCCG CAGGAAGAGG AACGGAGCGA GUCCCGCCGC GGCGCGAUUC 420
   CCUGAGCUGU GGGACGUGCA CCCAGGACUC GGCUCACACA UGCAGUUCGC UUUCCUGUUG 480
   GUGGGGGGAA CGCCGAUCGU GCGCAUCCGU CACCCCUCGC CGGCAGUGGG GGCUUGUGAA 540
   CCCCCAAACC UGACUGACUG GGCCAGUGUG CUGCAAAUUG GCAGAUCGAU GACCUAAGUG 600
   GAUCCGACUU GGUACCAUGA AUGGGCAGUG AGCCGGGGUU GCCUGGAGCC GUUCCUGCGU 660
   GGGUUCUCCC GUCUUCCGCU UUUUGUUGCC UUUUAUGGUU GUAUUACAAC UUAGUUCCUG 720
   CUCUGCAGAU UUUGUUGAGG UUUUUGCUUC UCCCAAGGUA GAUCUCGACC AGUCCCUCAA 780
   CGGGGUGUGG GGAGAACAGU CAUUUUUUUU UGAGAGAUCA UUUAACAUUU AAUGAAUAUU 840
   UAAUUAGAAG AUCUAAAUGA ACAUUGGAAA UUGUGUUCCU UUAAUGGUCA UCGGUUUAUG 900
   CCAGAGGUUA GAAGUUUCUU UUUUGAAAAA UUAGACCUUG GCGAUGACCU UGAGCAGUAG 960
   GAUAUAACCC CCACA 975
(2) INFORMATION ZU SEQ ID NO: 20:
   (i) SEQUENZ CHARAKTERISTIKA:
      (A) LÄNGE: 975 Basenpaare
      (B) ART: Nukleinsäure
      (C) STRANGFORM: Einzel
      (D) TOPOLOGIE: linear
   (ii) ART DES MOLEKÜLS: Genom-RNA
   (xi) SEQUENZBESCHREIBUNG: SEQ ID NO: 20:
   GGGCGAAUUG GCGGCCGCGG GUUGCGGAGG GUGGGCCUGG GAGGGGUGGU GGCCAUUUUU    60
   UGUCUAACCC UAACUGAGAA GGGCGUAGGC GCCGUGCUUU UGCUCCCCGC GCGCUGUUUU   120
   UCUCGCUGAC UUUCAGCGGG CGGAAAAGCC UCGGCCUGCC GCCUUCCACC GUUCAUUCUA   180
   GAGCAAACAA AAAAUGUCAG CUGCUGGCCC GUUCGCCUCC CGGGGACCUG CGGCGGGUCG   240
   CCUGCCCAGC CCCCGAACCC CGCCUGGAGC CGCGGUCGGC CCGGGGCUUC UCCGGAGGCA   300
   CCCACUGCCA CCGCGAAGAG UUGGGCUCUG UCAGCCGCGG GUCUCUCGGG GGCGAGGGCG   360
   AGGUUCACCG UUUCAGGCCG CAGGAAGAGG AACGGAGCGA GUCCCGCCGC GGCGCGAUUC   420
   CCUGAGCUGU GGGACGUGCA CCCAGGACUC GGCUCACACA UGCAGUUCGC UUUCCUGUUG   480
   GUGGGGGGAA CGCCGAUCGU GCGCAUCCGU CACCCCUCGC CGGCAGUGGG GGCUUGUGAA   540
   CCCCCAAACC UGACUGACUG GGCCAGUGUG CUGCAAAUUG GCAGAUCGAU UCCCGUCGCC   600
   AAAUCGAGCG GGUACCAUGA AUGGGCAGUG AGCCGGGGUU GCCUGGAGCC GUUCCUGCGU   660
   GGGUUCUCCC GUCUUCCGCU UUUUGUUGCC UUUUAUGGUU GUAUUACAAC UUAGUUCCUG   720
   CUCUGCAGAU UUUGUUGAGG UUUUUGCUUC UCCCAAGGUA GAUCUCGACC AGUCCCUCAA   780
   CGGGGUGUGG GGAGAACAGU CAUUUUUUUU UGAGAGAUCA UUUAACAUUU AAUGAAUAUU   840
   UAAUUAGAAG AUCUAAAUGA ACAUUGGAAA UUGUGUUCCU UUAAUGGUCA UCGGUUUAUG   900
   CCAGAGGUUA GAAGUUUCUU UUUUGAAAAA UUAGACCUUG GCGAUGACCU UGAGCAGUAG   960
   GAUAUAACCC CCACA                                                    975
(2) INFORMATION ZU SEQ ID NO: 21:
   (i) SEQUENZ CHARAKTERISTIKA:
      (A) LÄNGE: 975 Basenpaare
      (B) ART: Nukleinsäure
      (C) STRANGFORM: Einzel
      (D) TOPOLOGIE: linear
   (ii) ART DES MOLEKÜLS: Genom-RNA
   (xi) SEQUENZBESCHREIBUNG: SEQ ID NO: 21:
   GGGCGAAUUG GCGGCCGCGG GUUGCGGAGG GUGGGCCUGG GAGGGGUGGU GGCCAUUUUU    60
   UGUCUAACCC UAACUGAGAA GGGCGUAGGC GCCGUGCUUU UGCUCCCCGC GCGCUGUUUU   120
   UCUCGCUGAC UUUCAGCGGG CGGAAAAGCC UCGGCCUGCC GCCUUCCACC GUUCAUUCUA   180
   GAGCAAACAA AAAAUGUCAG CUGCUGGCCC GUUCGCCUCC CGGGGACCUG CGGCGGGUCG   240
   CCUGCCCAGC CCCCGAACCC CGCCUGGAGC CGCGGUCGGC CCGGGGCUUC UCCGGAGGCA   300
   CCCACUGCCA CCGCGAAGAG UUGGGCUCUG UCAGCCGCGG GUCUCUCGGG GGCGAGGGCG   360
   AGGUUCACCG UUUCAGGCCG CAGGAAGAGG AACGGAGCGA GUCCCGCCGC GGCGCGAUUC   420
   CCUGAGCUGU GGGACGUGCA CCCAGGACUC GGCUCACACA UGCAGUUCGC UUUCCUGUUG   480
   GUGGGGGGAA CGCCGAUCGU GCGCAUCCGU CACCCCUCGC CGGCAGUGGG GGCUUGUGAA   540
   CCCCCAAACC UGACUGACUG GGCCAGUGUG CUGCAAAUUG GCAGAUCGAU CGUCCAAUCG   600
   CUAUACUCUC GGUACCAUGA AUGGGCAGUG AGCCGGGGUU GCCUGGAGCC GUUCCUGCGU   660
   GGGUUCUCCC GUCUUCCGCU UUUUGUUGCC UUUUAUGGUU GUAUUACAAC UUAGUUCCUG   720
   CUCUGCAGAU UUUGUUGAGG UUUUUGCUUC UCCCAAGGUA GAUCUCGACC AGUCCCUCAA   780
   CGGGGUGUGG GGAGAACAGU CAUUUUUUUU UGAGAGAUCA UUUAACAUUU AAUGAAUAUU   840
   UAAUUAGAAG AUCUAAAUGA ACAUUGGAAA UUGUGUUCCU UUAAUGGUCA UCGGUUUAUG   900
   CCAGAGGUUA GAAGUUUCUU UUUUGAAAAA UUAGACCUUG GCGAUGACCU UGAGCAGUAG   960
   GAUAUAACCC CCACA                                                   975

## Beschreibung

Die Erfindung betrifft ein Verfahren zur Quantifizierung von Tumorzellen in einer Körperflüssigkeit, bei dem zuerst mit der zu untersuchenden Probe eine Reaktion durchgeführt wird, bei der die RNA-Komponente der Telomerase spezifisch amplifiziert, und anschließend die Menge an amplifizierter Nukleinsäure quantitativ bestimmt wird sowie dazu geeignete Testkits.

Nahezu alle soliden malignen Tumore haben das Potential zur Metastasenbildung. Der Prozeß der Metastasierung beinhaltet die Aussaat maligner Zellen als Mikrometastasen, zumeist auf dem Blut- bzw. Lymphweg in entfernte Organe und die Ausbildung autonomer Tochtergeschwülste. Das Ausmaß der Filiarisierung bestimmt die Prognose eines Tumorleidens.

Die Ansprüche von Tumorvorsorge- oder Nachsorgeprogrammen liegen in der Früherkennung von Primärtumoren bzw. eines Rezidivs oder einer Metastasierung noch bevor Metastasen klinisch manifest werden. Dieses Ziel kann bislang mit den verfügbaren apparativen Techniken nicht zufriedenstellend erfüllt werden, insbesondere gibt es immer noch eine diagnostische Grauzone zwischen zirkulierenden Tumorzellen und beginnender Metastasenbildung in Organen. Durch die Frühdiagnose zirkulierender maligner Zellen z. B. im peripheren Blut eines Tumornachsorgepatienten könnte frühzeitig, d.h. noch vor einer manifesten Organmetastasierung, eine möglicherweise kurative Immunmodulations- oder Polychemotherapie ergriffen werden. Die Quantifizierung der Metastasen im peripheren Blut vor und nach der Therapie stellt dabei eine wichtige Kontrolle dar.

In GB 2 260 811 wird beispielsweise ein Diagnoseverfahren zum Nachweis von malignen Tumoren vorgeschlagen, die mit normalen Zellen eines bestimmten Körpergewebes assoziiert sind, wobei die normalen Zellen mindestens ein für dieses Gewebe spezifisches Genprodukt bilden. Bei diesem Nachweisverfahren wird dem Patienten Körperflüssigkeit, beispielsweise Blut, entnommen, in der die Zellen bei einem gesunden Menschen normalerweise nicht vorkommen, und die mRNA des spezifischen Genproduktes amplifiziert und nachgewiesen. Als Beispiel wird die Tyrosinase zum Nachweis von Melanomzellen in peripherem Blut genannt. Nachteil dieser Methode ist jedoch, daß sie an gewebespezifische Genprodukte gebunden ist, eine Quantifizierung der Melanomzellen nicht ermöglicht und auch falsch-positive Ergebnisse liefert.

Kim et al. beschreiben die Ergebnisse eines Assays, mit dem Telomeraseaktivitäten in Tumorgeweben bestimmt werden konnte [Kim et al. (1994). Science 266: 2011]. Die Telomerase-Aktivität wurde mit einer Sensitivität von ca. 1 immortale Zelle/104 normale Zellen in 98 von 100 Krebszellkulturen bzw. 90 von 101 malignen Tumoren als auch in Keimgeweben nachgewiesen, nicht aber in 22 normalen somatischen Zellkulturen.

Die Telomerase ist ein neu beschriebenes Ribonukleoprotein mit reverser Transkriptaseaktivität [Shippen-Lentz et al. (1990), Science 247: 546], das als Matrize eine integrale RNA-Sequenz zur unabhängigen DNA-Synthese benutzt [Greider et al. (1989). Nature 337: 331], mit der neue telomere DNA an die Enden der Chromosomen synthetisiert werden. Telomere bestehen aus hoch konservierten (TTAGGG)n Tandemsequenzen von ca. 5-15 Kilobasen (kb) Länge/Zellgenom und haben die Aufgabe die Chromosomen an der Kernmembran zu stabilisieren und schützen die kodierende genomische DNA vor unkontrollierter Rekombination und Degradation [Mehle et al. (1994). Cancer Res 54: 236]. Während in den niederen Eukaryonten ein dynamisches Gleichgewicht zwischen Verkürzung der Chromosomenenden und der de novo Synthese von telomeren Sequenzen durch die Telomerase postuliert wird, zeigen normale humane somatische Zellen eine niedrige oder nicht nachweisbare Telomeraseaktivität. Darüberhinaus ist die Telomerase im Gegensatz zu anderen DNA Enzymen nicht wachstumsreguliert, denn keine der aktiv proliferierenden Zellkulturen zeigte nachweisbare Telomeraseaktivität. Einzig Keimzellen und fast alle Tumorzellinien [Ohyashiki et al. (1994). Cancer Genet Cytogenet 78: 64; Rogalla et al. (1994). Cancer Genet Cytogenet 77: 19; Schwartz et al. (1995). Cancer 75: 1094] und Tumorgewebe (Lunge, [Hiyama et al. (1995). Oncogene 10: 937; Shirotani et al. (1994). Lung Cancer 11: 29], Nieren [Mehle et al. (1994). Cancer Res 54: 236], Ovarien [Chadeneau et al. (1995). Cancer Res 55: 2533] und Blut [Counter et al. (1995). Blood 85: 2315]) zeigen meßbare Telomeraseaktivität und eine konstante Telomerlänge, die durch eine unendliche Zahl von Zellteilungen hindurch beibehalten wird. Daher kann die Aktivierung der Telomerase mit der damit verbundenen Stabilisierung der Telomerlängen als kritischer Schritt in Richtung Immortalisierung von somatischen Zellen gewertet werden.

Feng et al. gelang die Klonierung der integralen RNA-Sequenz der humanen Telomerase (hTR), die auf dem distalen Segment (q) von Chromosom 3 kodiert wird. Die Autoren konnten mittels kompetitiver Polymerase-Kettenreaktion (PCR) eine signifikante Erhöhung der Telomeraseexpression in Tumorgeweben sowie in den Keimgeweben gegenüber normalen somatischen Zellen belegen [Feng et al. (1995), Science 269: 1236]. Ein Antisense-Konstrukt der hTR-Sequenz verursachte den Zelltod (Apoptose) in tranfizierten HeLA-Zellen. Diese Daten belegen die stringente Repression der Telomerase in somatischen Geweben als auch die Tatsache, daß malignes Wachstum von der Präsenz immortaler Zellen und von der Aktivierung der Telomerase abhängig ist. Die WO 96/01835 offenbart die RNA-Komponente der Telomerase sowie deren Verwendung für diagnostische Zwecke.

Aufgabe der vorliegenden Erfindung war daher, ein Verfahren zu entwickeln, mit dem man Tumorzellen in einer Körperflüssigkeit quantitativ bestimmen kann.

Die Erfindung betrifft daher ein Verfahren zur Quantifizierung von Tumorzellen gemäß Anspruch 1 Hierbei wird mit der zu untersuchenden Probe eine Reaktion durchgeführt, bei der die RNA-Komponente der Telomerase spezifisch amplifiziert, und anschließend die Menge an amplifizierter Nukleinsäure quantitativ bestimmt wird sowie dazu geeignete Testkits. Als Körperflüssigkeit im Sinne der vorliegenden Erfindung versteht man Blut, insbesondere peripheres Blut.

Beispielsweise wird peripheres Blut durch Punktion einer Arterie, Vene oder Fingerkuppe dem Probanden entnommen und in einem RNA-Lysispuffer der beispielsweise Harnstoff oder vorzugsweise Guanidiniumisothiocyanat enthält, überführt, um eventuell vorhandene RNasen zu denaturieren und die Nukleinsäuren aus den Zellen freizusetzen [siehe z. B. Chomczynski et al. (1987) Anal. Biochem. 162, 156]. Die Isolierung der Nukleinsäuren aus dem stark salzhaltigen Medium des RNA-Lysispuffers kann beispielsweise mittels Siliciumdioxid-Partikel erfolgen, an die sämtliche Nukleinsäuren binden können [Boom et al. (1990) J. Clin. Microbiol., 29, 495]. Danach werden die Partikel mit geeignetem Puffer mehrmals gewaschen und die gebundenen Nukleinsäuren eluiert. Anschließend ist es vorteilhaft die in der Probe eventuell vorhandene genomische DNA mittels RNasefreier DNase in einem geeigneten Puffer zu hydrolysieren, damit bei der späteren Amplifizierung der RNA-Komponente der Telomerase keine falsch-positiven Ergebnisse bzw. ein zu großes Hintergrundrauschen durch falsche Amplifizierungssignale aufgrund eventuell noch vorhandener DNA entstehen. Anschließend erfolgt im allgemeinen eine Inaktivierung der DNase beispielsweise durch Phenolextraktion und/oder Hitzedenaturierung. Vor oder vorzugsweise nach Behandlung der Probe mit DNase kann vorteilhafterweise noch eine weitere Reinigung der in der Probe vorhandenen RNA beispielsweise mittels chromatographischer Methoden wie die Ionenaustausch-Chromatographie vorzugsweise an Kieselgel erfolgen.

Zur Kontrolle, ob noch eventuell störende genomische DNA in der Probe vorhanden ist, kann anschließend eine Amplifizierungsreaktion mit den unten beschriebenen Telomerase-spezifischen Oligonukleotid-Primern durchgeführt werden, wobei die in der Probe vorhandene RNA vorher nicht in cDNA durch eine reverse Transkriptionsreaktion umgeschrieben wird. Nur für den Fall, daß die Probe frei ist von Telomerasespezifischer DNA erfolgt keine Amplifikation mit der Folge, daß keine amplifizierte DNA gemessen werden kann.

Anschließend erfolgt eine Umschreibung der in der Probe vorhandenen RNA in cDNA im allgemeinen mit Hilfe der reversen Transkriptionsreaktion z. B. mit der AMV reversen Transkriptase. Das Verfahren ist allgemein bekannt und beispielsweise bei Sambrook et al., Molecular Cloning: A Laboratory Manual, New York Cold Spring Harbor Laboratory, 1989 beschrieben. In einer bevorzugten Ausführungsform der reversen Transkription kann auch eine thermostabile RNA-abhängige DNA Polymerase, wie in WO 90/07641 beschrieben, verwendet werden. Als Oligonukleotid-Primer für die reverse Transkriptase eignen sich beispielsweise vorteilhafterweise die unten beschriebenen Oligonukleotid-Primer oder Random Primer einer bestimmten Länge.

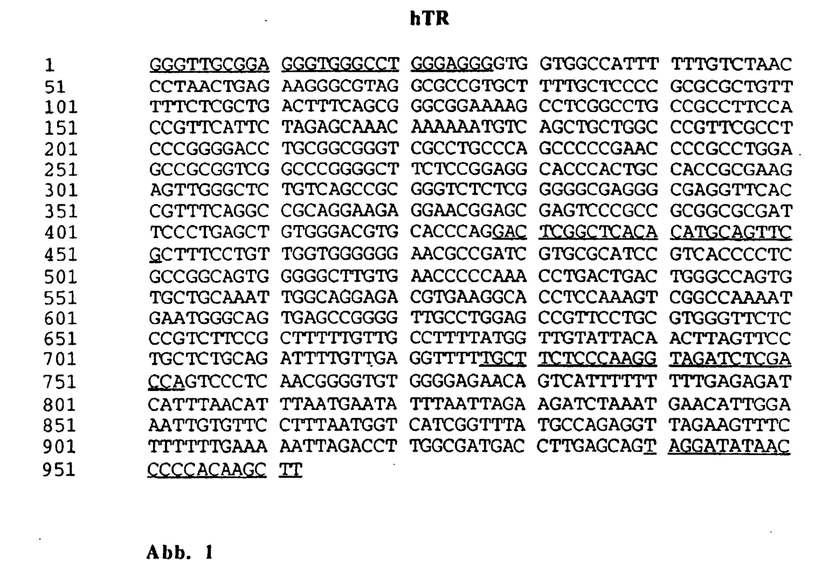

Die anschließende Amplifizierung kann beispielsweise mit einer DNA-Polymerase z. B. nach der Polymerase-Kettenreaktion (PCR) durchgeführt werden (siehe z. B. U.S. Patent Nos 4,683,195; 4,683,202; 4,965,188) oder vorzugsweise mit einer RNA-Polymerase nach z. B. der isothermalen Nukleinsäuresequenzbasierenden Amplifikation (NASBA). In jedem Fall benötigt man für die Amplifizierung spezifische Oligonukleotid-Primer, die von der zu amplifizierenden Nukleinsäure abgeleitet sind. Bei der vorliegenden Erfindung kann jeder beliebige Sequenzabschnitt der RNA-Komponente der Telomerase für die Synthese der Oligonukleotid-Primer verwendet werden. Vorzugsweise sind die Oligonukleotid-Primer ca. 20 bis ca. 30, vorzugsweise ca. 20 bis ca. 25 Nukleotide lang. Das Amplifikationsprodukt ist im allgemeinen ca. 100 bis ca. 2000 Basen, vorzugsweise ca. 200 bis ca. 1500 Basen, insbesondere ca. 300 bis ca. 350 Basen lang. Bei dem erfindungsgemäßen Verfahren sind insbesondere folgende Oligonukleotid-Primer bevorzugt, die aus der Sequenz gemäß Abb. 1 abgeleitet wurden:

5' GACTCGGCTC ACACATGCAG TTCGC 3' (TM1),

und/oder

5' CTGGTCGAGA TCTACCTTGG GAGAAGC 3' (TM2),

wobei TM1 und/oder TM2 gegebenenfalls zusätzlich eine Promotorsequenz für eine RNA-Polymerase enthalten können. Der Oligonukleotid-Primer TM1 entspricht dem 5'-Primer und TM2 dem 3'-Primer. Das Amplifikationsprodukt ist 327 bp lang. Die Primer können beispielsweise synthetisch nach der Triestermethode hergestellt werden [Matteucci et al., (1981), J. Am. Chem. Soc., 103, 3185-3191]. Als DNA-Polymerase kann beispielsweise eine nicht-thermostabile DNA-Polymerase wie die T4 DNA Polymerase, T7 DNA Polymerase, E. coli Polymerase I oder das Klenow Fragment von E. coli oder vorzugsweise eine thermostabile DNA-Polymerase wie die Taq-Polymerase (siehe z. B. U.S. Patent No. 4,889,818) verwendet werden.

Das allgemeine Prinzip der PCR-Reaktion besteht nun darin, daß während mehrerer sich wiederholender Reaktionszyklen die DNA hitzedenaturiert wird und in Anwesenheit geeigneter Oligonukleotid-Primer mit gegenseitiger Orientierung der Einzelstrang mit Hilfe der DNA-Polymerase zum Doppelstrang wieder ergänzt wird. Danach wird der Zyklus wiederholt bis sich genügend DNA zur Quantifizierung nach einer der unten beschriebenen Methoden gebildet hat. Im allgemeinen sind ca. 20 bis ca. 40 Zyklen, vorzugsweise ca. 30 bis ca. 35 Zyklen ausreichend.

Bei der NASBA-Methode (auch 3SR-System genannt) wird zumindest ein Oligonukleotid-Primer, vorzugsweise TM2 verwendet, der einen Promotor für die RNA Polymerase, vorzugsweise für die T7 RNA Polymerase enthält [siehe z. B. Guatelli et al. (1990) Proc. Natl. Acad. Sci. USA, 87, 1874-1878 oder Kievits et al. (1991), J. Virol. Methods, 35, 273-286]. Zuerst wird, wie oben bereits näher beschrieben, die RNA mit Hilfe eines der oben beschriebenen Oligonukleotid-Primer und einer reversen Transkriptase, vorzugsweise der AMV reversen Transkriptase, in cDNA umgeschrieben. Das Reaktionsprodukt ist ein RNA:DNA-Doppelstrang, dessen RNA-Komponente anschließend mittels einer RNase, vorzugsweise der RNase H, zu einem DNA-Einzelstrang abgebaut wird. Unter Verwendung eines der oben beschriebenen Oligonukleotid-Primer wird der DNA-Einzelstrang mittels einer DNA Polymerase zum DNA-Doppelstrang ergänzt. Als DNA Polymerase eignet sich vorzugsweise wiederum die AMV reverse Transkriptase, da diese Transkriptase nicht nur eine RNA-abhängige DNA Polymeraseaktivität, sondern auch eine DNA-abhängige DNA Polymeraseaktivität besitzt. Das Reaktionsprodukt ist ein DNA-Doppelstrang, der den Promotor für z. B. die T7 RNA Polymerase enthält. Anschließend synthetisiert die RNA Polymerase wieder einen sogenannten "antisense" RNA-Strang und der Zyklus kann von neuem beginnen. Im allgemeinen sind ca. 20 bis ca. 40 Zyklen, vorzugsweise ca. 30 bis ca. 35 Zyklen ausreichend, um genügend Amplifikationprodukt, vorzugsweise "antisense" RNA, für die anschließende Quantifizierung zu liefern.

Für die Quantifizierung der Amplifikationprodukte können diese beispielsweise mit Ethidiumbromid gefärbt und direkt z. B. in einem Agarose- oder Polyacrylamidgel nachgewiesen und quantifiziert werden. Es ist jedoch vorteilhaft, wenn die Amplifikationsprodukte bereits während der Amplifizierung markiert werden, weil dadurch eine höhere Sensitivität erreicht wird. Als Markierungen eignen sich beispielsweise radioaktive Markierungen, Biotinmarkierungen, Fluoreszenz- oder Elektrochemolumineszenz(ECL)markierungen. Die Markierungen tragen in der Regel die Nukleotide als Ausgangsstoffe für die Amplifizierung durch die DNA- oder RNA-Polymerase. Die radioaktiv markierten Amplifikationsprodukte (z. B. PCR- oder NASBA-Produkte) können durch Messung der Radioaktivität, die 3iotin-markierten Amplifikationsprodukte über einen Avidin- oder Streptavidin-tragenden Farbstoff, die fluoreszenzmarkierten Amplifikationsprodukte mit einem Fluorometer und die elektrochemolumineszenz-markierten Amplifikationsprodukte mit einem ECL-Detektor nachgewiesen werden. Die am meisten bevorzugte Nachweismethode ist jedoch die in vitro Hybridisierung mit einem bereits markiertem Oligonukleotid, das zu dem Amplifikationsprodukt komplementär ist. Das Oligonukleotid trägt im allgemeinen die oben beschriebenen Markierungen, wobei in Verbindung mit der NASBA-Amplifizierungsmethode die Hybridisierungsprobe eine Elektrochemolumineszenzmarkierung, vorzugsweise eine (Tris[2,2-bipyridin]ruthenium[II]-Komplexmarkierung trägt [siehe z. B. van Gemen et al. (1994), J. Virol. Methods, 49, 157-168].

Eine genaue und sensitive Quantifizierung der Amplifikationprodukte kann vorteilhafterweise dadurch erreicht werden, daß eine bestimmte Menge von einer oder mehreren bekannten Nukleinsäuren (Standard-Nukleinsäuren) co-amplifiziert werden [siehe z. B. van Gemen et al. (1993), J. Virol. Methods, 43, 177-188]. Hierbei wird zu den unbekannten Mengen der zu untersuchenden Probe eine unterschiedliche, jedoch genau bekannte Menge der co-amplifizierenden Standard-Nukleinsäure bzw. -Nukleinsäuren beispielsweise in Form einer Verdünnungreihe hinzugegeben und die Amplifikationprodukte der Probe und einer oder mehrerer co-amplifizierender Standard-Nukleinsäuren in unabhängigen Ansätzen quantitativ bestimmt. Ein Vergleich der Meßergebnisse ergibt die Menge der zu bestimmenden RNA-Komponente der Telomerase in der Probe.

Vorteilhafterweise wird die co-amplifizierende Standard-Nukleinsäure bzw. -Nukleinsäuren mit demselben Oligonukleotid-Primer amplifiziert wie die zu untersuchende Probe und die Amplifikationsprodukte haben auch im wesentlichen die gleiche Länge. Besonders bevorzugt haben die co-amplifizierenden Nukleinsäuren dieselbe Sequenz wie das Amplifikationsprodukt der zu bestimmenden Probe mit Ausnahme von ca. 20 Nukleinsäuren zwischen den Oligonukleotid-Primer-Bindungsstellen, die eine willkürliche, jedoch bekannte Sequenz tragen. Dadurch können das zu bestimmende Amplifikationsprodukt in der Probe von dem co-amplifizierenden Amplifikationsprodukt beispielsweise über eine Hybridisierung, wie z. B. bei Sambrook et al. (supra) näher beschrieben, mit entsprechend komplementären markierten Oligonukleotiden unabhängig voneinander quantifiziert werden. Insbesondere ist es vorteilhaft, wenn mehrere, vorzugsweise drei, verschiedene co-amplifizierende Nukleinsäuren in unterschiedlichen Konzentrationen der Probe zugesetzt werden, da hierdurch die Zahl der sonst einzeln durchzuführenden Amplifizierungsreaktionen reduziert werden kann [siehe van Gemen et al. (1994) J. Virol. Methods 49, 157-168]. Es ist auch insbesondere bevorzugt, die co-amplifizierenden Nukleinsäuren bereits zu dem oben beschriebenen RNA-Lysispuffer hinzuzugeben, da hierdurch der Einfluß möglicher Nukleinsäureverluste bei der nachfolgenden Aufarbeitung der Probe ausgeschlossen werden kann.

Als co-amplifizierende Standard-Nukleinsäuren gemäß der vorliegenden Erfindung eignen sich vorteilhafterweise RNA-Einzelstrang-Sequenzen, die durch in vitro Transkription beispielsweise mit der Sp6 oder T7 RNA Polymerase von Konstrukten hergestellt werden, die DNA oder cDNA der zu amplifizierenden Probe enthalten und die jeweils mit einem randomisierten Sequenzaustausch von beispielsweise ca. 10 bis ca. 30, vorzugsweise ca. 20 Nukleotiden ausgestattet sind.

Die Konstrukte bestehen bevorzugterweise aus einem Transkriptionsvektor mit einer Bindungstelle für die Sp6 oder T7 RNA Polymerase zwischen einer sogenannten "Multiple Cloning Site", in welcher die DNA oder cDNA der zu amplifizierenden Probe kloniert worden ist. Durch eine selektive Hydrolyse mit Restriktionsendonukleasen, vorzugsweise mit zwei verschiedenen Restriktionsendonukleasen, kann die klonierte Sequenz geöffnet und ein Stück einer bestimmten Länge herausgeschnitten und durch ein gleich langes Stück beispielsweise mit Hilfe der T4 Ligase ersetzt werden. Das klonierte Stück kann einen Sequenzaustausch beliebiger Länge, beispielsweise ca. 10 bis ca. 30, vorzugsweise ca. 20 Nukleinsäuren enthalten und liegt vorzugsweise zwischen den Oligonukleotid-Primer-Bindungsstellen. Diesen Vorgang kann man wiederholen, um an gleicher Stelle Insertionen mit anderen Nukleinsäure-Sequenzen vorzunehmen. Lassen sich keine geeigneten Schnittstellen finden, z. B. weil auch im Vektor geschnitten wird, müssen künstliche Schnittstellen geschaffen werden. Dies kann beispielsweise mittels rekombinanter PCR geschehen, die im wesentlichen bei Higuchi et al. [Higuchi, R. (1988). Nucleic Acid Res 16: 7351-7367; Higuchi, R. (1990). M. Innis A. et al. eds. San Diego, New York, Berkley, Boston, London, Sydney, Tokyo, Toronto, Academic Press, Inc. 177-183] und im experimentellen Teil der vorliegenden Erfindung beschrieben ist.

Eine bevorzugte Verwendung im Rahmen der Erfindung finden in vitro transkripierte RNA-Einzelstrang-Sequenzen von Konstrukten, welche
a) die gesamte cDNA der RNA Komponente der menschlichen Telomerase enthalten und
b) in welchen ein randomisierter Sequenzaustausch von ca. 20 Nukleotiden eingeführt worden ist.

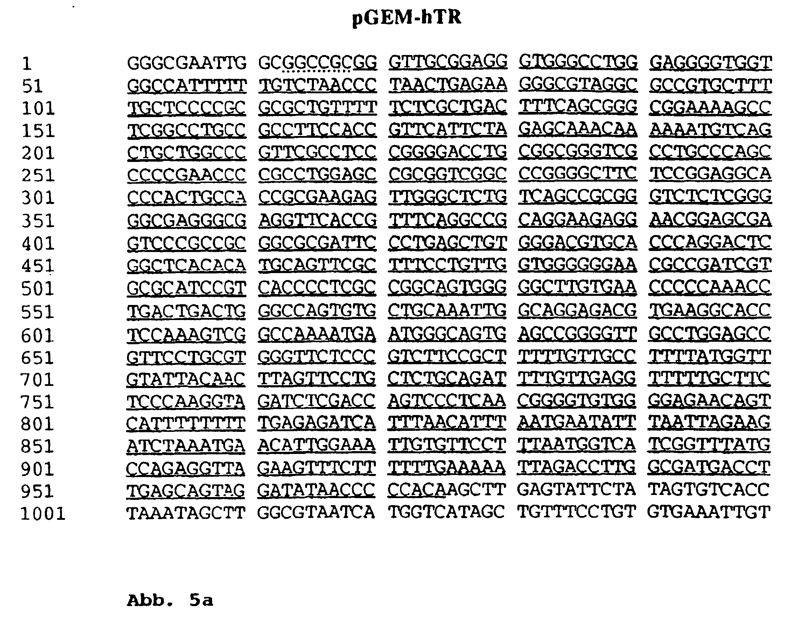

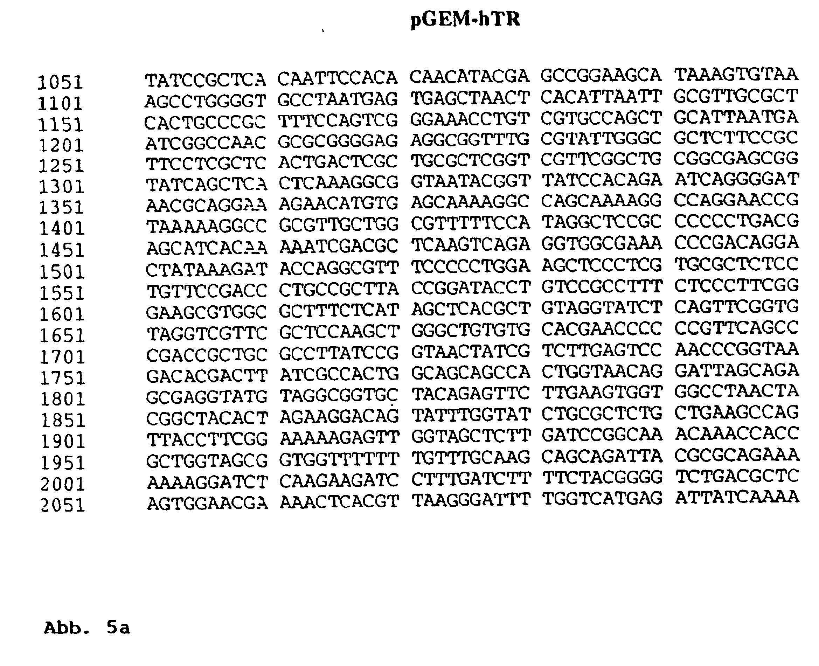

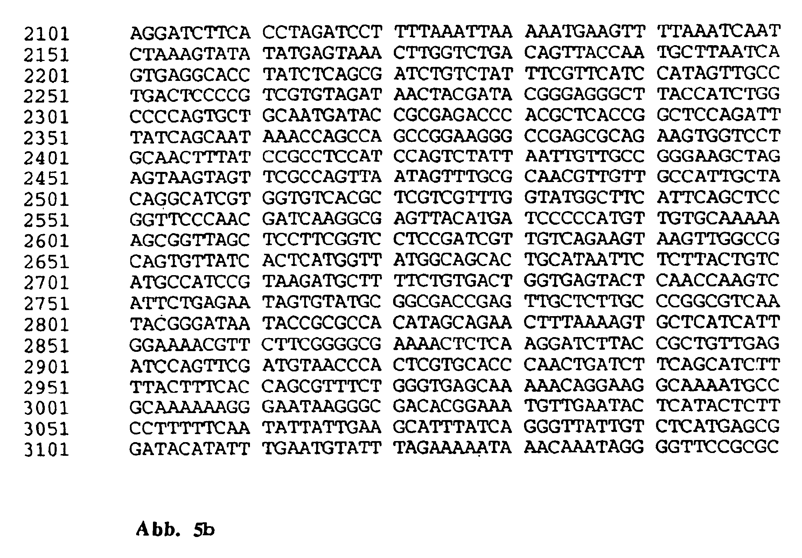

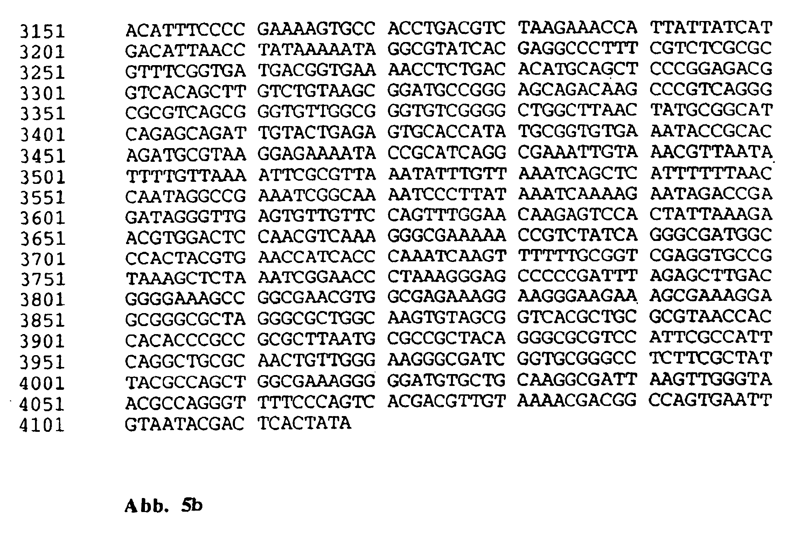

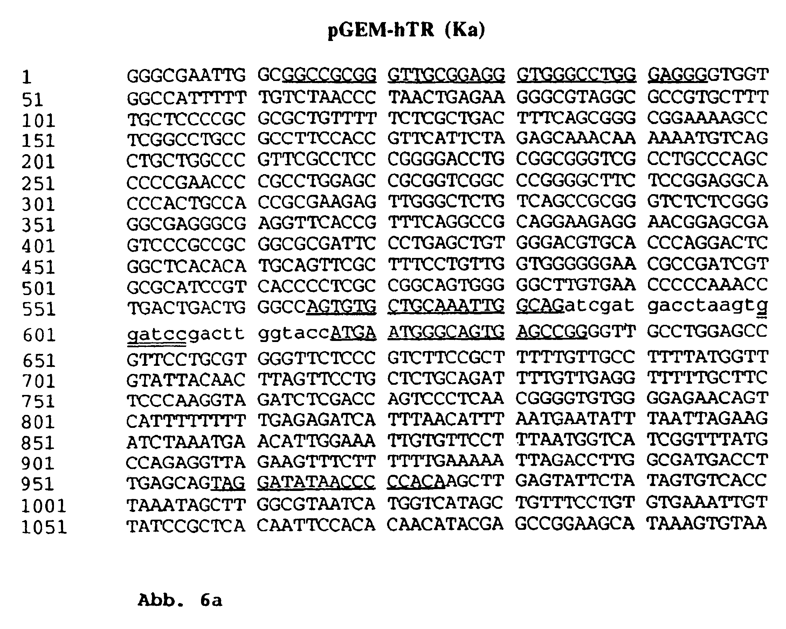

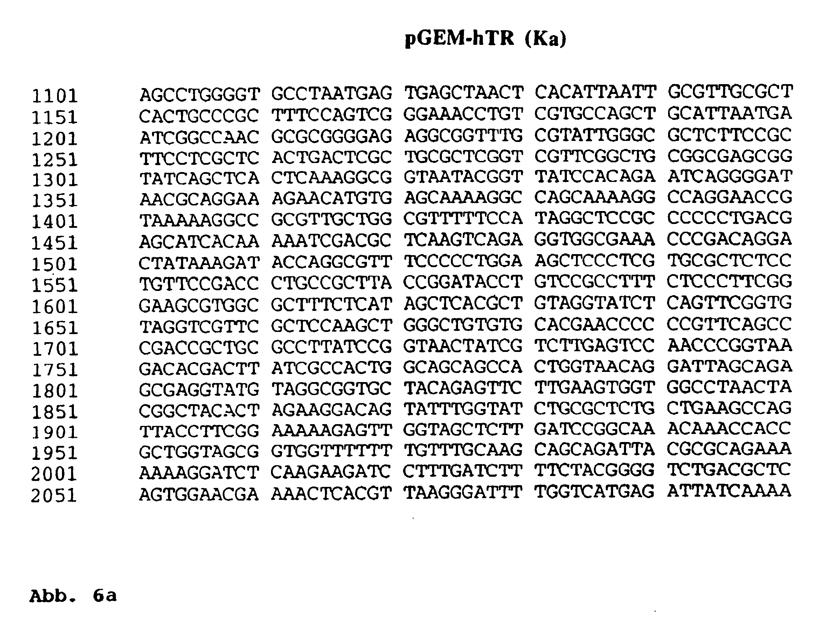

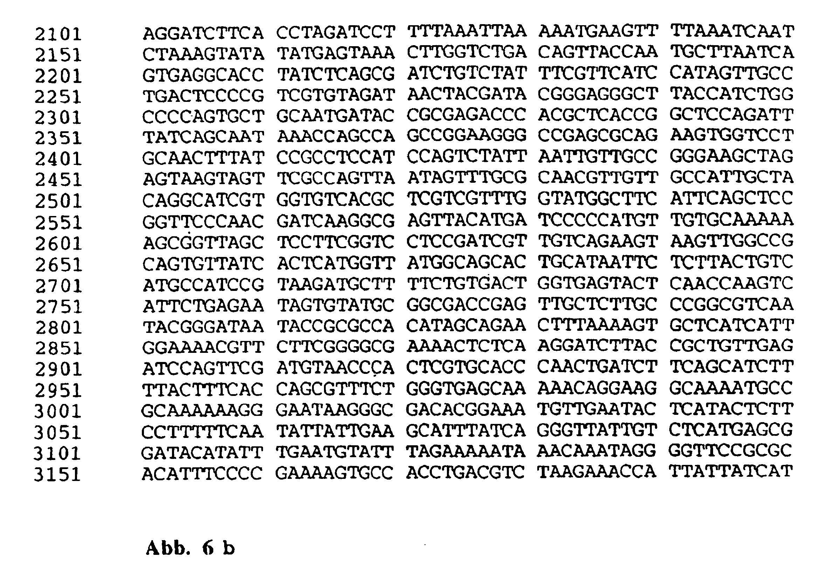

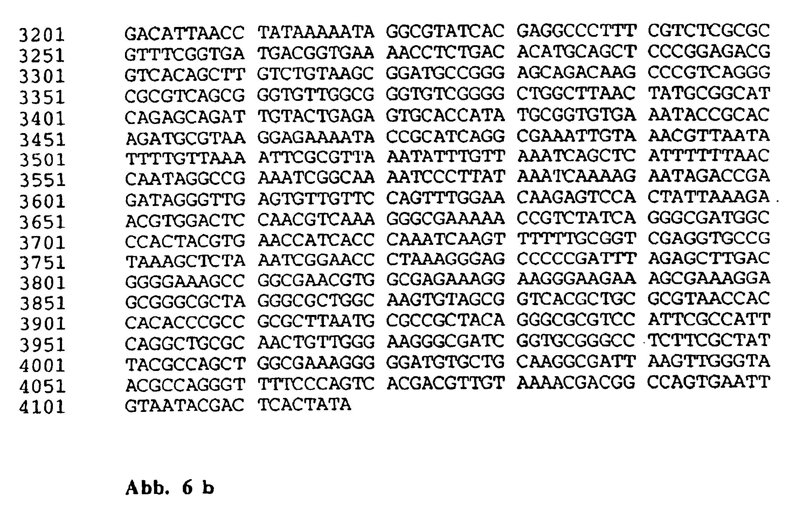

Die Konstrukte sind eine Ableitung der Konstrukte
pGEM-hTR gemäß Abb. 5a und 5b
pGEM-hTR(Ka) gemäß Abb. 6a und 6b.

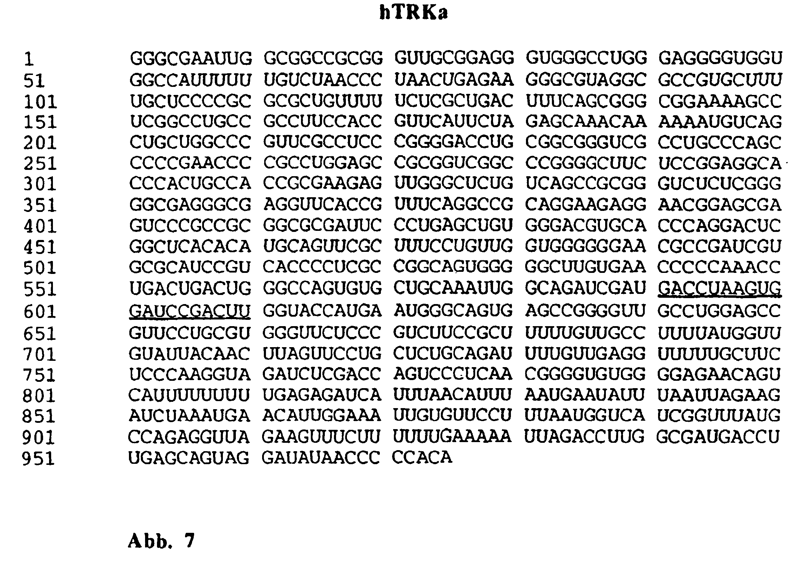

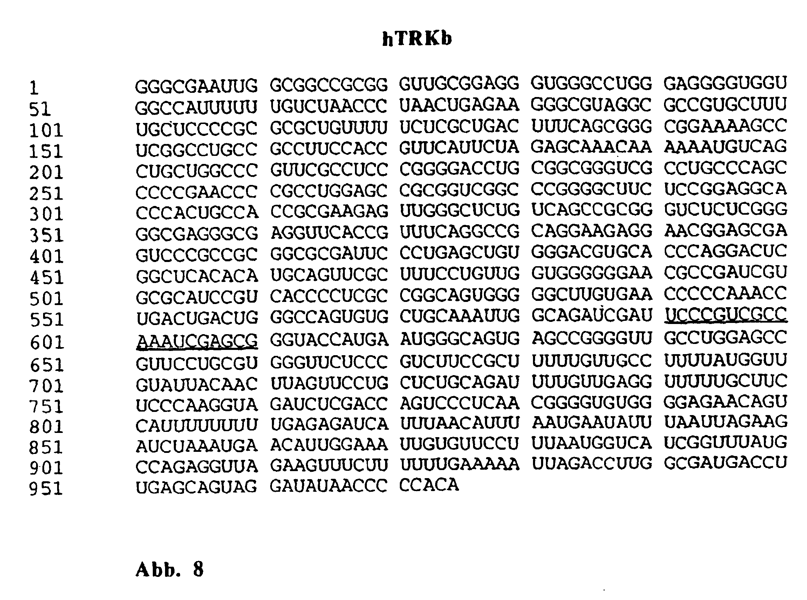

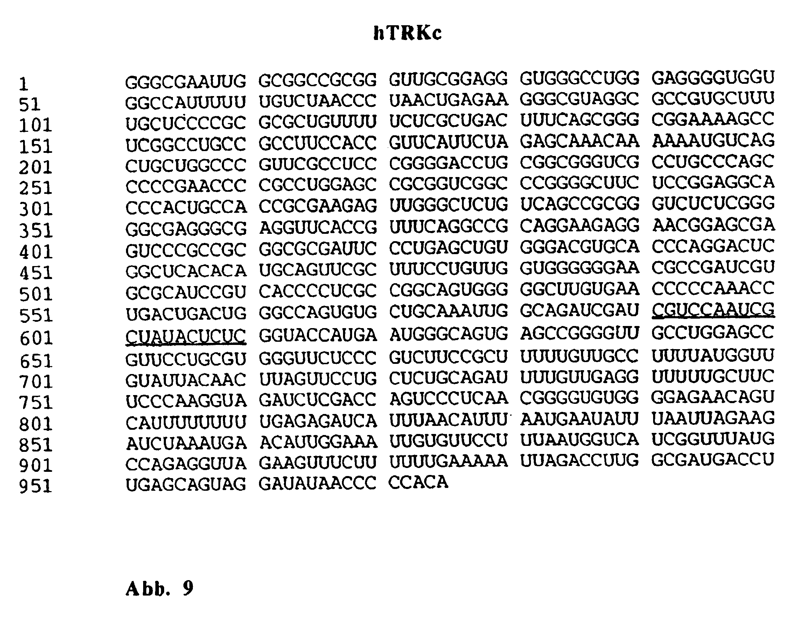

Durch in vitro Transkription der Konstrukte mit Sp6 RNA Polymerase können individuell 975 Basenpaar lange RNA-Standard-Nukleinsäuren hergestellt werden, mit der Sequenz:
(hTRKa) gemäß Abb. 7
(hTRKb) gemäß Abb. 8
(hTRKc) gemäß Abb. 9.

Die randomisierte Sequenz, worin sich die Standard-Nukleinsäuren von der Wildtyp-RNA voneinander unterscheiden, befindet sich bei diesem Beispiel in Position 591-610 gemäß Abb. 5a. Sie ist 20 Basenpaare lang.

Da sich die Standard-Nukleinsäuren untereinander und von der Wildtyp-Sequenz in diesem Beispiel nur durch eine randomisierte und bekannte 20 Basenpaar lange Sequenz unterscheiden, können die Amplifikationsprodukte der Standard-Nukleinsäuren und der Wildtyp-Sequenz durch komplementäre Bindung von markierten Oligonukleotiden in vier getrennten Ansätzen nachgewiesen werden. Als Oligonukleotide zum spezifischen Nachweis der amplifizierten cDNA der RNA Komponente der Telomerase (wt) und der Standard-Nukleinsäuren (hTRKa), (hTRKb) und (hTRKc) gemäß der vorliegenden Erfindung, eignen sich insbesondere folgende Sequenzen, die aus den Sequenzen gemäß Abb. 7-9 abgeleitet wurden:

5' CGACTTTGGA GGTGCCTTCA 3' O(wt)

5' AAGTCGGATC CACTTAGGTC 3' O(Ka)

5' CGCTCGATTT GGCGACGGGA 3' O(Kb)

5' GAGAGTATAG CGATTGGACG 3' O(Kc).

Zum Nachweis der amplifizierten "antisense" RNA, werden die entsprechenden reverse komplementären Sequenzen verwendet.

Danach werden die individuellen Amplifikationsmengen von Wildtyp- und den Standard-Nukleinsäuren bestimmt. Im Vergleich zu den unterschiedlichen Amplifikationsmengen der Standard-Nukleinsäuren bei bekannter Ausgangsmenge (z. B. hTRKa: 10², hTRKb: 10⁴ und hTRKc: 10⁶ Moleküle) läßt sich die unbekannte Ausgangsmenge der Wildtypsequenz errechnen. Daraus läßt sich dann auf die Anzahl der Metastasen pro abgenommener Körperflüssigkeit schließen.

Als interne positive Kontrolle des Verfahrens und der zu untersuchenden Probe kann zusätzlich eine Nukleinsäure amplifiziert und nachgewiesen werden, die im allgemeinen immer in einer Körperflüssigkeit vorkommt. Als geeignete Nukleinsäuren sind beispielsweise die mRNA kodierend für β-Globin oder für die Glycerinaldehydphosphatdehydrogenase (GAPDH) zu nennen (siehe z. B. GB 2 260 811), die immer in den Zellen der Körperflüssigkeit vorkommen. Geeignete Oligonukleotid-Primer für die humane ß-Globin mRNA sind beispielsweise Primer mit den Sequenzen:

5' ACCCAGAGGT TCTTTGAGTC 3'

und

5' TCTGATAGGC AGCCTGCACT 3',

wobei die Oligonukleotid-Primer gegebenenfalls zusätzlich eine Promotorsequenz für eine RNA-Polymerase enthalten können.

Zur Vermeidung bzw. Reduzierung falsch positiver Ergebnisse bzw. des sogenannten Hintergrundrauschens, das durch eventuell vorhandene Telomeraseaktivitäten von Nicht-Tumorzellen verursacht wird, ist es vorteilhaft vor der erfindungsgemäßen Untersuchung die entnommene Körperflüssigkeit aufzureinigen. Insbesondere sollen aus der zu untersuchenden Probe Stammzellen und/oder aktivierte Immunzellen abgereichert oder Tumorzellen angereichert werden. Da in der Regel die einzelnen Zellen spezifische Oberflächenmarker besitzen ist eine Abtrennung oder Anreicherung der Zellen über die Immunabsorption besonders vorteilhaft. Als Methoden eignen sich beispielsweise die magnetische (MACS) oder die fluoreszenzaktivierte (FACS) Zellsortierung [siehe z. B. Göttlinger & Radbruch (1993) mta, 8, 530-536, No. 5]. So können hämatopoetische Stammzellen beispielsweise über ihren Oberflächenmarker CD34 mittels MACS von der. Blutprobe abgetrennt werden [Kato & Radbruch (1993) Cytometry, 14, 384-392]. B-Zellen lassen sich beispielsweise über ihre Oberflächenmarker CD10, CD19 und/oder CD20 und T-Zellen über CD45RA und/oder CD7 abtrennen. Tumorzellen können über ihre spezifischen Tumormarker, z. B. CEA, angereichert werden. Neben MACS oder FACS eignen sich auch besonders an sogenannte käuflich erhältliche Magnetbeads (z. B. Dynabeads M450, Dynal Corp.) gebundene Antikörper gegen die spezifischen Oberflächenmarker zur Abreicherung oder Anreicherung der entsprechenden Zellen.

Allein oder in Verbindung mit den oben beschriebenen Reinigungsverfahren ist es auch besonders vorteilhaft die Menge an RNA-Komponente der Telomerase aus venösem Blut mit der Menge an RNA-Komponente der Telomerase aus arteriellem Blut zu vergleichen, da bei venöser Blutabnahme zum Zwecke der Tumorzellbestimmung etwa nur 20% aller Zellen gegenüber 100% der Zellen bei arterieller Blutabnahme nachweisbar sind [Koop, S. et al. (1995) Cancer Res. 55, 2520-2523]. Ebenso eignet sich ein Vergleich von Blut aus der Fingerkuppe mit venösem oder arteriellem Blut.

Die quantitative Bestimmung der RNA-Komponente der Telomerase in der Probe ermöglicht es, zu bestimmen, ob Tumorzellen, insbesondere Metastasen, vor allem Mikrometastasen, von malignen Tumoren in der Körperflüssigkeit enthalten sind und in welcher Menge. Dies ist insbesondere für die frühzeitige klinische Diagnose der Metastasenbildung von malignen Tumoren und für die Überwachung einer Tumortherapie von großem Nutzen. Als Tumorzellen können mit der vorliegenden Erfindung insbesondere Tumorzellen von Metastasen, vorzugsweise Mikrometastasen, von malignen Tumoren, vor allem Zellen metastasierender Tumore und/oder Neoplasien nachgewiesen werden, die beispielsweise von einem T-Zell-Lymphoblastom, T-Zell-Leukämiezellen, chronisch myeloische Leukämiezellen, akute lymphatische Leukämiezellen, chronisch lymphatische Leukämiezellen, Teratokarzinom, Melanom, Lungenkarzinom, Dickdarmkrebs, Brustkrebs, Leberzellkarzinom, Nierentumor, Nebennierentumor, Prostatakarzinom, Neuroblastom, Gehirntumor, kleines Lungenzellkarzinom, Rhabdomyosarkom, Leiomyosarkom und/oder Lymphom abstammen.

Weitere Gegenstände der vorliegenden Erfindung sind die Oligonukleotid-Primer mit der Sequenz

5' GACTCGGCTC ACACATGCAG TTCGC 3' (TM1),

5' CTGGTCGAGA TCTACCTTGG GAGAAGC 3' (TM2),

5' ATAAGAATGC GGCCGCGGGT TGCGGAGGGT GGGCCTGGGA GGG 3' (TMK1),

5' CCCAAGCTTG TGGGGGTTAT ATCCTA 3' (TMK2),

5' CGCGGATCCA CTTAGGTCAT CGATCTGCCA ATTTGCAGCA CACT 3' (TMK3)

und/oder

5' CGCGGATCCG ACTTGGTACC ATGAATGGGC AGTGAGCCGG 3' (TMK4),

wobei TM1 und/oder TM2 gegebenenfalls zusätzlich eine Promotorsequenz für eine RNA-Polymerase enthalten können;
sowie
ein Oligonukleotid mit der Sequenz

5' CCATCGATTC CCGTCGCCAA ATCGAGCGGG TACCCC 3' (Kb)

3' GGTAGCTAAG GGCAGCGGTT TAGCTCGCCC ATGGGG 5',

oder

5' CCATCGATCG TCCAATCGCT ATACTCTCGG TACCCC 3' (Kc)

3' GGTAGCTAGC AGGTTAGCGA TATGAGAGCC ATGGGG 5';

sowie
ein Nukleinsäurekonstrukt pGEM-hTR gemäß Abb. 5a und 5b oder ein Nukleinsäurekonstrukt pGEM-hTR(Ka) gemäß Abb. 6a und 6b;
sowie die RNA-Standard-Nukleinsäure zur Co-amplifikation mit der Sequenz:
(hTRKa) gemäß Abb. 7
(hTRKb) gemäß Abb. 8
(hTRKc) gemäß Abb. 9, bzw. die cDNAs davon,
sowie die Oligonukleotide zum Nachweis der amplifizierten cDNA der Wildtyp-Nukleinsäure und der cDNA der hTRKa-, hTRKb- und hTRKc-Standard-Nukleinsäuren mit der Sequenz:

5' CGACTTTGGA GGTGCCTTCA 3' O(wt)

5' AAGTCGGATC CACTTAGGTC 3' O(Ka)

5' CGCTCGATTT GGCGACGGGA 3' O(Kb)

5' GAGAGTATAG CGATTGGACG 3' O(Kc)

und die entsprechenden reverse komplementären Sequenzen der Oligonukleotide zum Nachweis der amplifizierten "antisense" RNA.

Ein anderer Gegenstand der Erfindung ist ein Kit zur Quantifizierung von Tumorzellen in Blut, insbesondere peripheres Blut, enthaltend
(a) Nukleinsäure bzw. Nukleinsäuren zur Co-amplifikation, und
(b) ein Oligonukleotid-Primerpaar zur spezifischen Amplifizierung von Telomerase-kodierender Nukleinsäure und der Nukleinsäure bzw. Nukleinsäuren gemäß (a), wobei vorzugsweise die RNA-Standard-Nukleinsäure zur Co-Amplifikation gemäß (a) folgende Sequenz hat oder haben:
   (hTRKa) gemäß Abb. 7
   (hTRKb) gemäß Abb. 8
   (hTRKc) gemäß Abb. 9,
und/oder das Oligonukleotid-Primerpaar vorzugsweise folgende Sequenzen:

5' GACTCGGCTC ACACATGCAG TTCGC 3' (TM1)

und/oder

5' CTGGTCGAGA TCTACCTTGG GAGAAGC 3' (TM2) hat,

wobei TM1 und/oder TM2 gegebenenfalls zusätzlich eine Promotorsequenz für eine RNA-Polymerase enthalten können.

Der erfindungsgemäße Kit kann auch zusätzlich, wie oben näher beschrieben, ein markiertes Oligonukleotid als Hybridisierungsprobe zum spezifischen Nachweis der amplifizierten cDNA der Wildtyp-Sequenz und/oder mehrere markierte Oligonukleotide als Hybridisierungsprobe zum spezifischen Nachweis der amplifizierten cDNA der Standard-Nukleinsäure bzw. -Nukleinsäuren enthalten. Darüberhinaus kann ein erfindungsgemäßer Kit für die PCR-Amplifikation zusätzlich die oben näher beschriebenen Enzyme, gegebenenfalls markierte Nukleotide und/oder geeignete Puffer enthalten, wie z. B. eine reverse Transkriptase, vorzugsweise eine AMV reverse Transkriptase, eine DNA-Polymerase, vorzugsweise eine Taq-Polymerase und/oder eine DNase und gegebenenfalls zur Abreicherung von Stammzellen und/oder aktivierten Immunzellen und/oder zur Anreicherung von Tumorzellen geeignete Mittel, wie oben bereits näher beschrieben.

Ein anderer erfindungsgemäßer Kit für die NASBA-Amplifikation kann ebenso neben der oben näher beschriebenen Standard-Nukleinsäuren ein markiertes Oligonukleotid als Hybridisierungsprobe zum spezifischen Nachweis der amplifizierten "antisense" RNA der Wildtyp-Seguenz und/oder mehrere markierte Oligonukleotide als Hybridisierungsprobe zum spezifischen Nachweis der amplifizierten "antisense" RNA der Standard-Nukleinsäure bzw. -Nukleinsäuren enthalten. Zusätzlich kann er ebenso die oben näher beschriebenen Enzyme, gegebenenfalls markierte Nukleotide und/oder geeignete Puffer, wie z. B. eine reverse Transkriptase, vorzugsweise eine AMV reverse Transkriptase, eine RNA-Polymerase, vorzugsweise eine T7 RNA-Polymerase, eine RNase H und/oder eine DNase enthalten, und gegebenenfalls zur Abreicherung von Stammzellen und/oder aktivierten Immunzellen und/oder zur Anreicherung von Tumorzellen geeignete Mittel, wie oben bereits näher beschrieben.

Die folgenden Beispiele und Figuren sollen die vorliegende Erfindung näher beschreiben, ohne sie jedoch darauf zu beschränken.

### Beschreibung der Figuren

Abb. 1 zeigt die RNA-Komponente der menschlichen Telomerase und die Position der entworfenen Oligonukleotid-Primer: 5'-Primer TM1 (Position 428-452) und 3'-Primer TM2 (Position 728-754) mit einem Amplifikationsprodukt von 327 Basenpaaren (bp) oder 5'-Primer TMK1 ([16 bp] + 1-27) und 3'-Primer TMK2 (Position 940-962 + [3 bp]) mit einem Amplifikationsprodukt von 981 bp. Eine Hydrolyse mit den Restriktionsendonukleasen NotI und HindIII ergibt das 962 bp Fragment hTR.

Abb. 2 zeigt eine PCR-Amplifikation an der cDNA von Tumorzellinien.
Banden 1: MT4, T-Zell-Lymphoblast-Zellinie, Banden 2: C8166, T-Zell-Leukämie-Zellinie, Banden 3: K562, Chronische Myeloische Leukämie- (CML-) Zellinie, Banden 4: Molt4, Akute Lymphatische Leukämie- (ALL-) Zellinie und Banden 5: Teratokarzinom-Zellinie; M: 100bp-Marker. hTR: RT-PCR mit den TM1- und TM2-Primern; hTR/ϕRT: PCR-Kontrollreaktion ohne reverse Transkriptions(RT)-Reaktion, GAPDH: RT-PCR-Kontrollreaktion mit Primern für die Glycerinaldehydphosphatdehydrogenase (GAPDH).

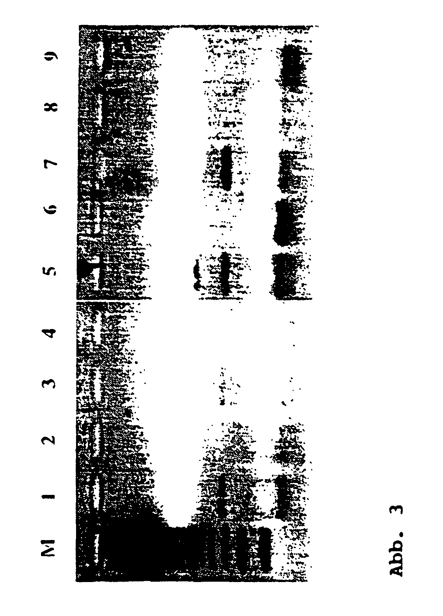

Abb. 3 zeigt eine PCR-Amplifikation mit den TM1- und TM2-Primern an der cDNA von Tumor- und gesunden Referenzgeweben. M: 100bp-Marker; Bande 1: Nieren-Karzinom, Bande 2: gesundes Nieregewebe; Bande 3: Schilddrüsen-Karzinom, Bande 4: gesundes Schildrüsengewebe; Bande 5: Mamma-Karzinom, gesundes Brustgewebe; Bande 7: Colon-Karzinom, Bande 8: gesundes Dickdarmgewebe; Bande 9: H₂O-Kontrollreaktion.

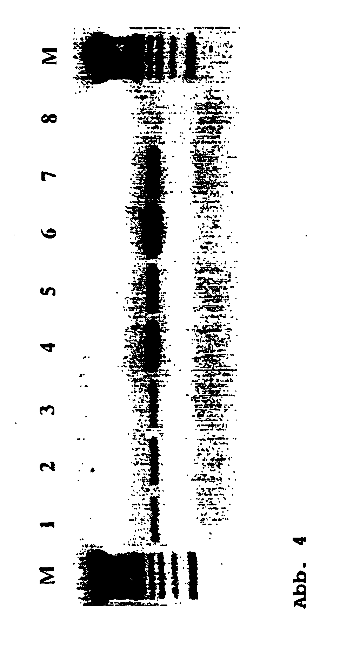

Abb. 4 zeigt eine PCR-Amplifikation mit den TM1- und TM2-Primern an der cDNA von peripherem Blut von normalen Probanden und Leukämiepatienten.
M: 100bp-Marker; Banden 1-3: gesunde Blutspender; Banden 4-8: Patienten mit akuter myeloischer Leukämie (AML); Bande 9: H₂O-Kontrollreaktion.

Abb. 5a und 5b zeigen das Konstrukt pGEM-hTR (4118 bp) mit dem Transkriptonsvektor pGEM-13Zf(+) und dem Fragment hTR(NotI/HindIII) gemäß Abb. 1, das die cDNA der RNA Komponente der menschlichen Telomerase (Basen 1-956: Position 12-975) enthält. Die Position der NotI-Addition (Position: 12-17) durch den Oligonukleotid-Primer TMK1 ist gepunktet gezeichnet.

Abb. 6a und 6b zeigen das Konstrukt pGEM-hTR(Ka) (4118 bp) mit der ClaI-BamHI-KpnI-Kassette (Position: 585-616) und die Positionen der entworfenen Oligonukleotid-Primer (5'-Primer TMK1: Position [16 bp] + 1-27, 3'-Primer TMK3: Position 565-605 + [24 bp]) mit einem Amplifikationsprodukt von 606 bp, (5'-Primer TMK4: Position 601-636 + [20 bp], 3'-Primer TMK2: Position 940-962 + [3 bp]) mit einem Amplifikationsprodukt von 387 bp. Eine Hydrolyse mit den Restriktionsendonukleasen NotI 387 bp. Eine Hydrolyse mit den Restriktionsendonukleasen NotI und BamHI bzw. BamHI und HindIII ergeben ein Produkt von 588 bzw. 375 bp. Die Ligierung der Fragmente in pGEM-13Zf(+) ergibt ein Produkt von 963 bp.

Abb. 7 zeigt die Standard-RNA hTRKa (975 bp) nach der in vitro Transkription mit Sp6 RNA Polymerase an dem mit HindIII linearisierten Konstrukt pGEM-hTRKa und die Position der randomisierten Sequenz von 20 bp (Position 591-610).

Abb. 8 zeigt die Standard-RNA hTRKb (975 bp) nach der in vitro Transkription mit Sp6 RNA Polymerase an dem mit HindIII linearisierten Konstrukt pGEM-hTRKb und die Position der randomisierten Sequenz von 20 bp (Position 591-610).

Abb. 9 zeigt die Standard-RNA hTRKc (975 bp) nach der in vitro Transkription mit Sp6 RNA Polymerase an dem mit HindIII linearisierten Konstrukt pGEM-hTRKc und die Position der randomisierten Sequenz von 20 bp (Position 591-610).

### BEISPIELE

wenn nicht anders vermerkt, wurden die folgenden Beispiele nach Standardverfahren, wie z. B. bei Sambrook, J. et al. (1989) supra beschrieben, oder nach den Herstellerangaben der verwendeten Kits bzw. Enzyme durchgeführt.

### 1. Kultivierung und Isolierung von peripherem Blut, Gewebe und Zellen

Tumorzellinien wie MT4 (T-Zell-Lymphoblast), C8166 (T-Zell-Leukämie), K562 (Chronisch Myeloische Leukämie (CML)), Molt4 (Akute Lymphatische Leukämie (ALL)) und Teratokarzinom wurden gemäß den Vorgaben der ATCC (American Tissue Culture Collection), in Kultur genommen. Venöses Spenderblut von Leukämiepatienten (akute myeloische Leukämie) und gesunden Kontrollpersonen wurde mittels Punktion einer Unterarmvene in EDTA-Monovetten® (Saarsted) abgenommen. Menschliches Tumor- und gesundes Referenzgewebe wurden nach Entnahme direkt in flüssigem Stickstoff schockgefroren und bei -70°C gelagert.

### 2. Isolierung von zellulärer RNA

Die Isolierung von totaler zellulärer RNA erfolgte nach Standardverfahren [Chomczynski et al. (1987) Anal Biochem 162, 156; Sambrook, J. et al. (1989). Cold Spring Harbor, New York, USA, Cold Spring Harbor Laboratory Press]. Peripheres Blut wurde sofort nach Abnahme in RNA-Lysispuffer (4 M Guanidinium Isothiocyanat; 0.1 M Tris-HCl, pH 7.5; 1% Mercaptoethanol) überführt. Gewebe und Zellen wurden mit einem Dispergiergerät Ultra-Turrax T25 (Laborreaktor-Systeme, IKA) in RNA-Lysisbuffer homogenisiert. Die Gemische wurden entweder sofort weiterverarbeitet oder bei -70°C gelagert.

### 3. Reverse Transkription und Polymerase-Kettenreaktion (RT-PCR)

Die RT-PCR wurde mit dem GeneAmp® RNA-PCR-Kit (Perkin Elmer) nach Vorgaben des Herstellers durchgeführt. Aliquote der isolierten totalen RNA von peripherem Blut, Zellinien und Geweben wurden jeweils zuvor mit 20U DNAse (Boehringer, Mannheim) in 10 µl Ansätzen (in 50 mM KCl; 10 mM Tris-HCl, pH 8.3 und 2.5 mM MgCl₂) bei 37°C für 60 Minuten hydrolysiert und anschließend die DNAse für 30 Minuten bei 95°C inaktiviert. Zur vollständigen Aufreinigung der RNA von Proteinen und DNA-Fragmenten wurden die DNAse-Hydrolysate jeweils über eine Kieselgelmatrix (RNeasy®, Qiagen) erneut aufgereinigt und photometrisch gemessen.

Die beiden Oligonukleotid-Primer:

TM1 5' GACTCGGCTC ACACATGCAG TTCGC 3'

TM2 5' CTGGTCGAGA TCTACCTTGG GAGAAGC 3'

wurden nach der von Feng et al. veröffentlichten Sequenz der RNA-Komponente der menschlichen Telomerase (Feng, J. et al. (1995). Science 269: 1236-41) entworfen (Abb. 1) und mit einem Applied Biosystem 380A Synthesizer synthetisiert. Die Spezifizität der TM1- und TM2-Primer wurde mittels Computer gestützter Homologieanalyse an den Nukleinsäuresequenzen in den GenBank-, EMBL-, DDBJ- und PDB-Datenbanken mittels BLASTN 1.4.9 MP [Altschul, S. F. et al. (1990). J Mol Biol 215: 403-410] überprüft.

Zur Abstimmung der Amplifikationsmengen wurden für jedes Experiment gleiche RNA-Mengen für die RT-Reaktion eingesetzt. Um eine Kontamination der RNA Präparationen mit genomischer DNA auszuschließen, wurde jede mit DNase hydrolysierte RNA-haltige Probe zuerst der unten beschriebenen PCR unterzogen und auf Amplifikation hin überprüft. Die RNA-haltige Probe, bei der kein Amplifikationsprodukt nachweisbar war, wurde für die folgenden cDNA-Synthese- und PCR-Schritte eingesetzt. Als interne Standardkontrolle wurden Oligonukleotid-Primer für die GAPDH eingesetzt.

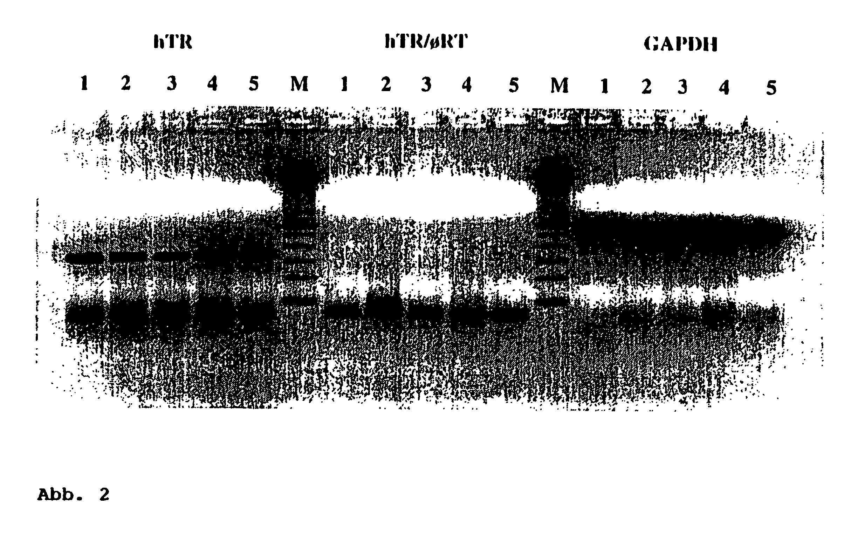

Die PCR wurde an 5 µl der cDNA-Reaktion bzw. an einer 1:50 Verdünnung der gewonnenen Plasmid-DNA nach folgendem Programm durchgeführt: (95°C: 2 Minuten, vorwärmen); (94°C: 30 Sekunden, 60°C: 30 Sekunden, 72°C: 30 Sekunden, 35 Zyklen); (72°C: 7 Minuten, finale Extension). Die Amplifikationsprodukte wurden gelelektrophoretisch auf 1.5%igem TAE Agarosegel aufgetrennt, mit Ethidiumbromid gefärbt und unter UV-Licht visualisiert und fotodokumentiert (siehe Abb. 2-4).

### 4. Herstellung der RNA-Standard-Nukleinsäuren hTRKa, hTRKb und hTRKc

Die verwendeten Enzyme, wie Sp6 RNA Polymerase, T4 Ligase bzw. Restriktionsendonukleasen u.a. von Boehringer Mannheim, Biolabs bzw. Promega wurden nach Herstellervorgaben verwendet. Die zur Klonierung bestimmten PCR-Amplifikationsprodukte wurden gelelektrophoretisch auf 1.5% TAE Agarose aufgetrennt und eluiert (Qiagen). Die Aufreinigung der Restriktionshydrolysate geschah durch Phenol-Chlorophorm-Extraktion und Fällung in Salz und Ethanol oder durch DNS-Reinigung (Qiagen). Die Konstrukte wurden durch Ligierung der Fragmente in die korrespondierenden Schnittstellen des Klonierungs- und Transkriptionsvektors pGEM-13Zf(+) mit Hilfe der T4 Ligase kloniert. Dieser Vektor erlaubt die in vitro Transkription von klonierten Fragmenten durch den wahlweisen Einsatz von Sp6- oder T7 RNA Polymerasen. Kompetente Bakterien (XL-1Blue, Stratagen) wurden mittels Elektroporation (BioRad) transformiert. Plasmid DNA wurde mittels Plasmid Reinigungs Kits (Qiagen) gereinigt. Positive Klone wurden unter Verwendung von vektor- oder sequenzspezifischen Oligonukleotid-Primern mit der PCR validiert. Eine Sequenzvalidierung der Konstrukte erfolgte durch semiautomatische Sequenzanalyse.

Das Konstrukt pGEM-hTR (Abb. 5a und 5b) wurde als Ausgangskonstrukt für die Konstrukte pGEM-hTR(Ka) (Abb. 6a und 6b), pGEM-hTR(Kb) und pGEM-hTR(Kc) geschaffen. pGEM-hTR(Ka) unterscheidet sich von pGEM-hTR durch einen randomisierten Sequenzaustausch in Position 585-616. Die Konstrukte pGEM-hTRKb und pGEM-hTRKc unterscheiden sich von pGEM-hTR durch einen randomisierten Sequenzaustausch in Position 587-615. Die Konstrukte wurden zur in vitro Transkription mit Sp6 RNA Polymerase der Standard-RNA: hTRKa (Abb. 7), hTR Kb (Abb. 8) und hTRKc (Abb. 9), verwendet. Zur Bildung des Konstrukts pGEM-hTR wurde die cDNA der RNA Komponente der menschlichen Telomerase hTR (Abb. 1) in die NotI und HindIII-Schnittstellen von pGEM-13Zf(+) kloniert. Dies wurde dadurch erreicht, daß mit den folgenden Oligonukleotid-Primern die aus der Sequenz hTR (Abb. 1) abgeleitet wurden

5' ATAAGAATGC GGCCGCGGGT TGCGGAGGGT GGGCCTGGGA GGG 3' (TMK1)

5' CCCAAGCTTG TGGGGGTTAT ATCCTA 3' (TMK2)

eine RT-PCR an der bereits gewonnenen RNA aus Tumorzellen oder -linien unter den oben beschriebenen Bedingungen durchgeführt wurde. Der Oligonukleotid-Primer TMK1 (Position 1-27, Abb.1) enthält 5' zusätzlich eine Verlängerung von 16 bp und eine NotI-Schnittstelle, der Oligonukleotid-Primer TMK2 (Position 940-962, Abb.1) zusätzlich eine Verlängerung von 3 bp und eine HindIII-Schnittstelle. Das Primerpaar TMK1 und TMK2 amplifiziert ein 979 bp Fragment. Nach einer Restriktionshydrolyse mit NotI und HindIII wurde das 963 bp Fragment hTR(NotI-HindIII) (Position 1-957, Abb. 5) in die korrespondierenden Schnittstellen (Position 12 bzw. 38) von pGEM-13Zf(+) kloniert und das 4118 bp Konstrukt pGEM-hTR geschaffen. Die Konstruktion von pGEM-hTR(Ka) wurde dadurch erreicht, daß im Konstrukt pGEM-hTR (Position 585-616) eine 32 bp Sequenz mit einer 32 bp ClaI-BamHI-KpnI-Kassette:

5' ATCGATGACC TAAGTGGATC CGACTTGGTA CC 3'

3' TAGCTACTGG ATTCACCTAG GCTGAACCAT GG 5'

ausgetauscht wurde. Dieser Austausch wurde durch rekombinante PCR durchgeführt und ist eine Abwandlung der von Higuchi et al. beschriebenen Verfahren [Higuchi, R. (1988). Nucleic Acid Res 16: 7351-7367; Higuchi, R. (1990). M. Innis A. et al. eds. San Diego, New York, Berkley, Boston, London, Sydney, Tokyo, Toronto, Academic Press, Inc. 177-183]. In einem ersten Schritt wurden zwei unabhängige PCR-Reaktionen an pGEM-hTR durchgeführt:

Die 1. PCR-Reaktion erfolgte mit den folgenden Oligonukleotid-Primern, die aus der Sequenz hTR (Abb. 1) abgeleitet wurden:

5' ATAAGAATGC GGCCGCGGGT TGCGGAGGGT GGGCCTGGGA GGG 3' (TMK1)

5' CGCGGATCCA CTTAGGTCAT CGATCTGCCA ATTTGCAGCA CACT 3' (TMK3)

Der Oligonukleotid-Primer TMK3 (Position 565-605, Abb. 6) enthält 5' zusätzlich eine Verlängerung von 24 bp mit einer BamHI- und ClaI-Schnittstelle und kodiert 21 bp der ClaI-BamHI-KpnI-Kassette. Das Amplifikat aus der 1. PCR-Reaktion ergibt das 5' Fragment von 606 bp und wurde mit NotI und BamHI zu einem 588 bp 5'-Fragment verdaut.

Die 2. PCR-Reaktion erfolgte mit den folgenden Oligonukleotid-Primern, die aus der Sequenz hTR (Abb. 1) abgeleitet wurden:

5' CGCGGATCCG ACTTGGTACC ATGAATGGGC AGTGAGCCGG 3' (TMK4)

5' CCCAAGCTTG TGGGGGTTAT ATCCTA 3' (TMK2).

Der Oligonukleotid-Primer TMK4 (Position 599-618, Abb 6) enthält 5' zusätzlich eine Verlängerung von 20 bp mit einer BamHI- und KpnI-Schnittstelle und kodiert 17 bp der ClaI-BamHI-KpnI-Kassette. Das Amplifikat aus der 2. PCR-Reaktion ergibt das 3' Fragment von 387 bp und wurde mit BamHI und HindIII zu einem 375 bp 3'-Fragment hydrolysiert. Mit T4 Ligase wurden die BamHI-Schnittstellen der 5'- und 3'-Fragmente miteinander zu dem 963 bp NotI-HindIII-Fragment verbunden, in die korrespondierenden Schnittstellen (Position 12 bzw. 38) von pGEM-13Zf(+) kloniert und das 4118 bp Konstrukt pGEM-hTR(Ka) geschaffen (Abb. 6).Die Konstruktion von pGEM-hTR(Kb) und pGEM-hTR(Kc) wurde dadurch erreicht, daß eine 29 bp Sequenz im Konstrukt pGEM-hTR(Ka) (Position 587-615) mit einer randomisierten Sequenz von 29 bp ausgetauscht wurde. Nach einem selektiven Restriktionsverdau mit ClaI und KpnI an dem Konstrukt pGEM-hTR(Ka) und den nachfolgenden Oligonukleotiden Kb und Kc wurden in zwei getrennten T4 Ligase-Reaktionen das ClaI-KpnI-Fragment der Oligonukleotide Kb und Kc in die korrespondierenden Schnittstellen von pGEM-hTR(Ka) kloniert und die 4118 bp Konstrukte pGEM-hTR(Kb) und pGEM-hTR(Kc) geschaffen.

In vitro RNA wurde mit Sp6 RNA Polymerase von pGEM-hTR(Ka) (hTRKa, Abb 7), pGEM-hTR(Kb) (hTRKb, Abb 8), und pGEM-hTR(Kc) (hTRKc, Abb 9) in einer Länge von 975 bp geschaffen. Die weitere Aufbereitung der RNA, wie DNAse Verdau, Reinigung und Kalibrierung erfolgte nach Standardmethoden.

### 5. Ergebnisse

Die Untersuchungen an Tumorzellinien ergaben, daß die RNA-Komponente der menschlichen Telomerase in unterschiedlichen Mengen in allen Tumorzellinien bei gleicher Amplifikationsmenge der GAPDH-Kontrollreaktion nachweisbar war (Abb. 2). Eine Kontamination mit genomischer DNA konnte durch Kontrollreaktion ohne Zugabe von reverser Transkriptase jeweils ausgeschlossen werden.

Die vergleichenden Untersuchungen an Tumorgewebe und gesundem Gewebe ergaben, daß die RNA-Komponente der menschlichen Telomerase eindeutig in Tumorgeweben jedoch nicht in gesunden Referenzgeweben nachgewiesen werden konnte (Abb.3). Die unterschiedliche Intensität der Amplifikationsprodukte kann mit der individuellen Güte der gewonnenen RNA aus den Tumorgeweben erklärt werden.

Die Untersuchungen mit venösem Blut ergaben, daß im Blut gesunder Probanden und von Leukämiepatienten unterschiedlich hohe Telomeraseaktivitäten nachgewiesen werden konnte, wobei im Vergleich zu den Krebspatienten bei den Kontrollpersonen deutlich weniger Telomeraseaktivitäten gefunden wurde (Abb. 4).

Die in vitro Transkription mittels Sp6 RNA Polymerase an den Konstrukten pGEM-hTR(Ka), pGEM-hTR(Kb) und pGEM-hTR(Kc) ergab jeweils das gewünschte RNA-Transkript hTRKa, hTRKb und hTRKc mit einer Länge von 975 Basen.

## Patentansprüche

1. Verfahren zur Quantifizierung von Tumorzellen in einer Körperflüssigkeit, bei dem
(a) mit der zu untersuchenden Probe eine Reaktion durchgeführt wird, bei der die RNA-Komponente der Telomerase spezifisch amplifiziert wird, und
(b) die Menge an amplifizierter Nukleinsäure quantitativ bestimmt wird, **dadurch gekennzeichnet, daß** es sich bei der zu untersuchenden Probe um Blut handelt, und daß aus der zu untersuchenden Blutprobe Stammzellen und/oder aktivierte lmmunzellen vorzugsweise mittels tmmunabsorption abgereichert werden und/oder, daß aus der zu untersuchenden Blutprobe die Tumorzellen vorzugsweise mittels Immunabsorption angereichert werden.

2. Verfahren nach Anspruch 1, **dadurch gekennzeichnet, daß** vor , der Amplifizierungsreaktion mit der zu untersuchenden Probe eine reverse Transkriptionsreaktion durchgeführt wird, bei der die in der Probe enthaltene RNA in cDNA umgeschrieben wird.

3. Verfahren nach Anspruch 1 oder 2, **dadurch gekennzeichnet, daß** mit der zu untersuchenden Probe vor der Umschreibung der RNA in cDNA eine DNase-Reaktion durchgeführt wird.

4. Verfahren nach einem der Ansprüche 1-3, **dadurch gekennzeichnet, daß** die zu untersuchende Probe vorzugsweise über eine lonenaustauschchromatographie, insbesondere über Kieselgel gereinigt wird.

5. Verfahren nach einem der Ansprüche 1-4, **dadurch gekennzeichnet, daß** zur quantitativen Bestimmung der Tetomerase-kodierenden Nukleinsäure mindestens eine, vorzugsweise drei Standard-Nukleinsäuren co-amplifiziert werden, die in unterschiedlichen Konzentrationen zu der zu untersuchenden Probe dazugegeben werden.

6. Verfahren nach Anspruch 5, **dadurch gekennzeichnet, daß** die co-amplifizierende(n) Standard-Nukleinsäure(n) die Sequenz gemäß Abb. 7, 8 und/oder 9 besitzen.

7. Verfahren nach einem der Ansprüche 1-6, **dadurch gekennzeichnet, daß** das Amplifizierungsprodukt entweder direkt oder über eine Markierung vorzugsweise über eine radioaktive Markierung, eine Biotin-Markierung, eine Fluoreszenz-Markierung oder eine Elektrochemoluinineszenz-Markierung quantifiziert wird.

8. Verfahren nach einem der Ansprüche 1-6, **dadurch gekennzeichnet, daß** das Amplifizierungsprodukt über eine Hybridisierung mit einem markierten Oligonukleotid nachgewiesen wird, wobei die Markierung vorzugsweise eine radioaktive Markierung, eine Biotin-Markierung, eine Fluoreszenz-Markierung oder eine Elektrochemolumineszenz-Markierung ist.

9. Verfahren nach einem der Ansprüche 5-8, **dadurch gekennzeichnet, daß** zur Quantifizierung der zu bestimmenden Telomerase-kodierenden Nukleinsäure die Menge der co-amplifizierten Nukleinsäure bzw. Nukleinsäuren mit der Menge der Telomerase-kodierenden Nukleinsäure verglichen wird.

10. Verfahren nach einem der Ansprüche 1-9, **dadurch gekennzeichnet, daß** es sich bei der zu untersuchenden Probe um peripheres Blut handelt, und daß als positive Kontrolle mit der zu untersuchenden Probe eine Reaktion durchgeführt wird, bei der eine im peripheren Blut vorkommende Nukleinsäure, vorzugsweise die mRNA kodierend für β-Globin oder Glycerinaldehydphosphatdehydrogenase, spezifisch amplifiziert und nachgewiesen wird.

11. Verfahren nach Anspruch 1 oder nach einem der Ansprüche 3-10, **dadurch gekennzeichnet, daß** als negative Kontrollen vor der Amplifizierungsreaktion mit der zu untersuchenden Probe keine reverse Transkriptionsreaktion durchgeführt wird und/oder anstelle der Körperflüssigkeit Wasser eingesetzt wird.

12. Verfahren nach einem der Ansprüche 1-11, **dadurch gekennzeichnet, daß** zur Amplifizierung folgende Oligonukleotid-Primer verwendet werden:
5' GACTCGGCTC ACACATGCAG TTCGC 3' (TM1)
und/oder
5' CTGGTCGAGA TCTACCTTGG GAGAAGC 3' (TM2),
wobei TM1 und/oder TM2 gegebenenfalls eine Promotorsequenz für eine RNA-Polymerase enthält.

13. Verfahren nach einem der Ansprüche 1-12, **durch gekennzeichnet**, **daß** zur Amplifizierung eine DNA-Polymerase oder eine RNA-Polymerase verwendet wird.

14. Verfahren nach einem der Ansprüche 1-13, **dadurch gekennzeichnet, daß** im Falle der Amplifizierung mit der DNA-Polymerase die Polymerase-Ketten-Reaktion (PCR) und im Falle der Amplifizierung mit der RNA-Polymerase die isothermale Nukleinsäuresequenz-basierende Amplifikation (NASBA) durchgeführt wird.

15. Verfahren nach einem der Ansprüche 1-14, **dadurch gekennzeichnet, daß** es sich bei der zu untersuchenden Probe um Blut handelt, und daß bei dem genannten Verfahren zum einen eine venöse Blutprobe und zum anderen eine arterielle Blutprobe untersucht wird und die Ergebnisse miteinander verglichen werden.

16. Verfahren nach einem der Ansprüche 1-15, **dadurch gekennzeichnet, daß** es sich bei der zu untersuchenden Probe um Blut handelt, und daß bei dem genannten Verfahren zum einen eine Blutprobe aus der Fingerkuppe und zum anderen eine venöse oder arterielle Blutprobe untersucht wird und die Ergebnisse miteinander verglichen werden.

17. Verfahren nach einem der Ansprüche 1-16, **dadurch gekennzeichnet, daß** die Tumorzellen von Metastasen, vorzugsweise Mikrometastasen, von malignen Tumoren stammen.

18. Verfahren nach einem der Ansprüche 1-17, **dadurch gekennzeichnet, daß** die Tumorzellen aus einer Gruppe von Zellen metastasierender Tumoren und/oder Neoplasien ausgewählt werden, die von einem T-Zell-Lymphoblastom, T-Zell-Leukämiezellen, chronisch myeloische Leukämiezellen, akute lymphatische Leukämiezellen, chronisch lymphatische Leukämiezellen, Teratokarzinom, Melanom, Lungenkarzinom, Dickdarmkrebs, Brustkrebs, Leberzellkarzinom, Nierentumor, Nebennierentumor, Prostatakarzinom, Neuroblastom, Gehirntumor, Rhabdomyosarkom, Leiomyosarkom und/oder Lymphom abstammen.

19. Oligonukleotid-Primer mit der Sequenz
5' GACTCGGCTC ACACATGCAG TTCGC 3' (TM1);
5' CTGGTCGAGA TCTACCTTGG GAGAAGC 3' (TM2);
5' ATAAGAATGC GGCCGCGGGT TGCGGAGGGT GGGCCTGGGA GGG 3' (TMK1) ;
5' CCCAAGCTTG TGGGGGTTAT ATCCTA 3' (TMK2) ;
5' CGCGGATCCA CTTAGGTCAT CGATCTGCCA ATTTGCAGCA CACT 3' (TMK3) ;
und/oder
5' CGCGGATCCG ACTTGGTACC ATGAATGGGC AGTGAGCCGG 3' (TMK4).

20. Oligonukleotid mit der Sequenz
5' CCATCGATTC CCGTCGCCAA ATCGAGCGGG TACCCC 3' (Kb)
3' GGTAGCTAAG GGCAGCGGTT TAGCTCGCCC ATGGGG 5',
und/oder
5' CCATCGATCG TCCAATCGCT ATACTCTCGG TACCCC 3' (Kc)
3' GGTAGCTAGC AGGTTAGCGA TATGAGAGCC ATGGGG 5'.

21. Standard-Nukleinsäure zur Co-amplifikation mit der Sequenz gemäß Abb. 7, 8 oder 9, oder die entsprechende cDNA davon.

22. Nukleinsäurekonstrukt pGEM-hTR gemäß Abb. 5a und 5b.

23. Nukleinsäurekonstrukt pGEM-hTR(Ka) gemäß Abb. 6a und 6b.

24. Kit zur Quantifizierung von Tumorzellen in einer Körperflüssigkeit enthaltend
(a) Standard-Nukleinsäure bzw. Standard-Nukleinsäuren zur Co-amplifikation, und
(b) ein Oligonukleotid-Primerpaar zur spezifischen Amplifizierung von Telomerase-kodierender Nukleinsäure und der Nukleinsäure bzw. Nukleinsäuren gemäß (a), wobei die Standard-Nukleinsäure bzw. Standard-Nukleinsäuren gemäß (a) eine Sequenz gemäß Abb. 7, 8 und/oder 9 hat bzw. haben, **dadurch gekennzeichnet, daß** das Oligonukleotid-Primerpaar gemäß (b) folgende Sequenzen hat:
5' GACTCGGCTC ACACATGCAG TTCGC 3' (TM1)
und
5' CTGGTCGAGA TCTACCTTGG GAGAAGC 3' (TM2),
wobei TM1 und/oder TM2 gegebenenfalls eine Promotorsequenz für eine RNA-Polymerase enthält.

25. Kit nach Anspruch 24, **durch gekennzeichnet**, **daß** er zusätzlich ein markiertes Oligonukleotid zum Nachweis der amplifizierten Nukleinsäure der zu bestimmenden Probe und/oder ein oder mehrere markierte Oligonukleotide zum Nachweis der co-amplifizierten Standard-Nukleinsäure bzw. Standard-Nukleinsäuren enthält, insbesondere die Oligonukleotide mit den Sequenzen:
5' CGACTTTGGA GGTGCCTTCA 3' O(wt)
5' AAGTCGGATC CACTTAGGTC 3' O(Ka)
5' CGCTCGATTT GGCGACGGGA 3' O(Kb)
5' GAGAGTATAG CGATTGGACG 3' O(Kc),
und die entsprechenden reverse komplementären Sequenzen davon.

26. Kit nach einem der Ansprüche 24-25, **dadurch gekennzeichnet, daß** er zusätzlich eine reverse Transkriptase, eine DNA-Polymerase, vorzugsweise eine Taq-Polymerase, eine DNase und/oder geeignete Puffer und gegebenenfalls markierte Nukleotide und gegebenenfalls zur Abreicherung von Stammzellen und/oder aktivierten Immunzellen und/oder zur Anreicherung von Tumorzellen geeignete Mittel anhält.

27. Kit nach einem der Ansprüche 24-26, **dadurch gekennzeichnet, daß** er zusätzlich eine reverse Transkriptase, eine RNA-Polymerase, vorzugsweise eine T7 RNA-Polymerase, eine RNase H, eine DNase und/oder geeignete Puffer und gegebenenfalls markierte Nukleotide und gegebenenfalls zur Abreicherung von Stammzellen und/oder aktivierten lmmunzellen und/oder zur Anreicherung von Tumorzellen geeignete Mittel enthält.

## Claims

1. A method for the quantification of tumor cells in a body fluid, which comprises
(a) carrying out a reaction with the sample to be investigated, in which reaction the RNA component of telomerase is specifically amplified, and
(b) determining quantitatively the amount of amplified nucleic acid, wherein the sample to be investigated is blood, and wherein the blood sample to be investigated is depleted in stem cells and/or activated immune cells, preferably by immunoabsorption and/or wherein the tumor cells from the blood sample to be investigated are concentrated, preferably by immunoabsorption.

2. A method as claimed in claim 1, wherein a reverse transcription reaction in which the RNA contained in the sample is transcribed into cDNA is carried out before the amplification reaction with the sample to be investigated.

3. The method as claimed in claim 1 or 2, wherein a DNase reaction is carried out with the sample to be investigated before the transcription of the RNA into cDNA.

4. The method as claimed in any of claims 1-3, wherein the sample to be investigated is purified, preferably by an ion exchange chromatography, in particular on silica gel.

5. The method as claimed in any of claims 1-4, wherein, for quantitative determination of the telomerase-encoding nucleic acid, at east on, preferably three, standard nucleic acids are coamplified and are added in different concentrations to the sample to be investigated.

6. The method as claimed in claim 5, wherein the coamplifying standard nucleic acid(s) have the sequence shown in Fig. 7, 8 and/or 9.

7. The method as claimed in any of claims 1-6, wherein the amplification product is quantified either directly or via a label, preferably via a radioactive label, a biotin label, a fluorescent label or an electrochemoluminescent label.

8. The method as claimed in any of claims 1-6, wherein the amplification product is detected via a hybridization with a labeled oligonucleotide, where the label is preferably a radioactive label, a biotin label, a fluorescent label or an electrochemoluminescent label.

9. The method as claimed in any of claims 5-8, wherein, to quantify the telomerase-encoding nucleic acid to be determined, the amount of coamplified nucleic acid or nucleic acids is compared with the amount of telomerase-encoding nucleic acid.

10. The method as claimed in any of claims 1-9, wherein the sample to be investigated is peripheral blood, and wherein a reaction is carried out with the sample to be investigated as positive control, in which a nucleic acid which occurs in peripheral blood, preferably the mRNA coding for β-globin or glyceraldehyde-phosphate dehydrogenase, is specifically amplified and detected.

11. The method as claimed in claim 1 or any of claim 3-10, wherein, as negative controls, no reverse transcription reaction is carried out before the amplification reaction with the sample to be investigated and/or water is employed in place of the body fluid.

12. The method as claimed in any of claims 1-11, wherein the following oligonucleotide primers are used for the amplification:
5' GACTCGGCTC ACACATGCAG TTCGC 3' (TM1)
and/or
5' CTGGTCGAGA TCTACCTTGG GAGAAGC 3' (TM2),
where TM1 and/or TM2 comprises where appropriate a promoter sequence for an RNA polymerase.

13. The method as claimed in any of claims 1-12, wherein a DNA polymerase or an RNA polymerase is used for the amplification.

14. The method as claimed in any of claims 1-13, wherein, in the case of amplification with DNA polymerase, the polymerase chain reaction (PCR) is carried out and, in the case of amplification with RNA polymerase, the isothermal nucleic acid sequence-based amplification (NASBA) is carried out.

15. The method as claimed in any of claims 1-14, wherein the sample to be investigated is blood, and wherein there is an investigation in said method of, on the one hand, a venous blood sample and, on the other hand, an arterial blood sample, and the results are compared with one another.

16. The method as claimed in any of claims 1-15, wherein the sample to be investigated is blood, and wherein there is an investigation in said method of, on the one hand, a blood sample from the finger pad and, on the other hand, a venous or arterial blood sample, ad the results are compared with one another.

17. The method as claimed in any of claims 1-16, wherein the tumor cells are derived from metastases, preferably micrometastases, of malignant tumors.

18. The method as claimed in any of claims 1-17, wherein the tumor cells are selected from a group of cells of metastasizing tumors and/or neoplasms which are derived from a T-cell lymphoblastoma, T-cell leukemia cells, chronic myeloid leukemia cells, acute lymphatic leukemia cells, chronic lymphatic leukemia cells, teratocarcinoma, melanoma, carcinoma of the lung, large intestine cancer, breast cancer, hepatocellular carcinoma, kidney tumor, adrenal tumor, prostate carcinoma, neuroblastoma, brain tumor, rhabdomyosarcoma, leiomyosarcoma and/or lymphoma.

19. An oligonucleotide primer with the sequence
5' GACTCGGCTC ACACATGCAG TTCGC 3' (TM1);
5' CTGGTCGAGA TCTACCTTGG GAGAAGC 3' (TM2);
5' ATAAGAATGC GGCCGCGGGT TGCGGAGGGT GGGCCTGGGA GGG 3' (TMK1);
5' CCCAAGCTTG TGGGGGTTAT ATCCTA 3' (TMK2);
5' CGCGGATCCA CTTAGGTCAT CGATCTGCCA ATTTGCAGCA CACT 3' (TMK3);
and/or
5' CGCGGATCCG ACTTGGTACC ATGAATGGGC AGTGAGCCGG 3' (TMK4).

20. An oligonucleotide with the sequence
5' CCATCGATTC CCGTCGCCAA ATCGAGCGGG TACCCC 3' (KB)
3' GGTAGCTAAG GGCAGCGGTT TAGCTCGCCC ATGGGG 5',
and/or
5' CCATCGATCG TCCAATCGCT ATACTCTCGG TACCCC 3' (Kc)
3' GGTAGCTAGC AGGTTAGCGA TATGAGAGCC ATGGGG 5'.

21. A standard nucleic acid for coamplification with the sequence shown in Fig. 7, 8 or 9, or the corresponding cDNA thereof.

22. A nucleic acid construct pGEM-hTR as shown in Figs. 5a and 5b.

23. A nucleic acid construct pGEM-hTR (Ka) as shown in Figs. 6a and 6b.

24. A kit for the quantification of tumor cells in a body fluid, comprising
(a) standard nucleic acid or standard nucleic acids for coamplification, and
(b) an oligonucleotide primer pair for specific amplification of telomerase-encoding nucleic acids specified in (a), wherein the oilgonucleodtide primer pair specified in (b) has the following sequences:
5' GACTCGGCTC ACACATGCAG TTCGC 3' (TM1)
and
5' CTGGTCGAGA TCTACCTTGG GAGAAGC 3' (TM2),
where TM1 and/or TM2 comprises where appropriate a promoter sequence for an RNA polymerase.

25. A kit as claimed in claim 24, which additionally comprises a labeled oligonucleotide for detecting the amplified nucleic acid of the sample to be determined and/or one or more labeled oligonucleotides for detecting the coamplified standard nucleic acid or standard nucleic acids, in particular the oligonucleotides with the sequences:
5' CGACTTTGGA GGTGCCTTCA 3' 0(wt)
5' AAGTCGGATC CACTTAGGTC 3' 0(Ka)
5' CGCTCGATTT GGCGACGGGA 3' 0(Kb)
5' GAGAGTATAG CGSTTGGSCG 3' 0(Kc),
and the corresponding reverse complementary sequences thereof.

26. A kit as claimed in any of claims 24-25, which additionally comprises a reverse transcriptase, a DNA polymerase, preferably a Taq polymerase, a DNase and/or suitable buffers and, where appropriate, labeled nucleotides and, where appropriate, means suitable for the depletion of stem cells and/or activated immune cells and/or for the concentration of tumor cells.

27. A kit as claimed in any of claims 24-26, which additionally comprises a reverse transcriptase, an RNA polymerase, preferably a T7 RNA polymerase, an RNase H, a DNase and/or suitable buffers and, where appropriate, labeled nucleotides and, where appropriate, means suitable for the depletion of stem cells and/or activated immune cells and/or for the concentration of tumor cells.

## Revendications

1. Procédé pour quantifier le nombre de cellules tumorales dans un volume de fluide corporel, **caractérisé en ce que** l'on
(a) soumet l'échantillon destiné à l'analyse à une réaction lors de laquelle on réalise une amplification spécifique du composant ARN de la télomérase et
(b) détermine la quantité de l'acide nucléique amplifié, **caractérisé en ce que** l'échantillon destiné à l'analyse est constitué de sang, et **en ce que** l'on diminue, de préférence au moyen d'une immunoabsorption, la concentration des cellules souche et/ou les cellules immunitaires activées dans l'échantillon de sang destiné à l'analyse, et/ou **en ce que** l'on augmente, de préférence au moyen d'une immunoabsorption, la concentration des cellules tumorales dans l'échantillon de sang destiné à l'analyse.

2. Procédé selon la revendication 1, **caractérisé en ce que** l'on soumet l'échantillon destiné à l'analyse, avant de réaliser la réaction d'amplification spécifique, à une réaction de transcription inverse lors de laquelle on transcrit l'ARN contenu dans l'échantillon pour obtenir l'ADNc.

3. Procédé selon la revendication 1 ou 2, **caractérisé en ce que** l'on soumet l'échantillon destiné à l'analyse à une réaction de l'ADNase, avant de procéder à la transcription de l'ARN en ADNc.

4. Procédé selon l'une quelconque des revendications 1 à 3, **caractérisé en ce que** l'on procède à une purification de l'échantillon destiné à l'analyse, de préférence au moyen d'une chromatographie par échange d'ions, en particulier sur gel de silice.

5. Procédé selon l'une quelconque des revendications 1 à 4, **caractérisé en ce que** l'on procède à la détermination quantitative de l'acide nucléique qui code pour la télomérase, au moyen d'une co-amplification d'au moins un, de préférence de trois acides nucléiques étalons que l'on ajoute, à des concentrations différentes, à l'échantillon destiné à l'analyse.

6. Procédé selon la revendication 5, **caractérisé en ce que** le ou les acides nucléiques étalons aptes à la co-amplification possèdent la séquence selon figure 7, 8 et/ou 9.

7. Procédé selon l'une quelconque des revendications 1 à 6, **caractérisé en ce que** l'on réalise la quantification du produit de l'amplification, soit directement, soit au moyen d'un marquage, de préférence au moyen d'un marquage radioactif, un marquage par la biotine, un marquage pour une fluorescence ou un marquage pour une luminescence électrochimique.

8. Procédé selon l'une quelconque des revendications 1 à 6, **caractérisé en ce que** l'on détecte le produit de l'amplification au moyen d'une hybridation avec un oligonucléotide marqué, ce marquage étant de préférence un marquage radioactif, un marquage par la biotine, un marquage pour une fluorescence ou un marquage pour une luminescence électrochimique.

9. Procédé selon l'une quelconque des revendications 5 à 8, **caractérisé en ce que** l'on compare la quantité de l'acide nucléique ou des acides nucléiques que l'on a soumis auparavant à la co-amplification et la quantité de l'acide nucléique qui code pour la télomérase, afin de quantifier l'acide nucléique destiné au dosage et qui code pour la télomérase.

10. Procédé selon l'une quelconque des revendications 1 à 9, **caractérisé en ce que** l'échantillon destiné à l'analyse est constitué de sang périphérique, et **en ce que** l'on réalise, en tant que contrôle positif, une réaction lors de laquelle l'on procède à une amplification spécifique d'un acide nucléique présent dans le sang périphérique, de préférence l'ARNm qui code pour la β-globine ou la glycéraldéhydphosphate déhydrogénase, et à sa detection.

11. Procédé selon la revendication 1 ou selon l'une quelconque des revendications 3 à 10, **caractérisé en ce que** l'on réalise, en tant que témoins négatifs, la réaction d'amplification de l'échantillon sans réaction de transcription inverse préalable et/ou **en ce que** l'on met en oeuvre de l'eau à la place du volume de fluide corporel.

12. Procédé selon l'une quelconque des revendications 1 à 11, **caractérisé en ce que** l'on met en oeuvre pour procéder à l'amplification, les amorces oligonucléotide suivantes:
5' GACTCGGCTC ACACATGCAG TTCGC 3' (TM1)
et/ou
5' CTGGTCGAGA TCTACCTTGG GAGAAGC 3' (TM2),
où TM1 et/ou TM2 contiennent éventuellement une séquence amorce pour une ARN polymérase.

13. Procédé selon l'une quelconque des revendications 1 à 12, **caractérisé en ce que** l'on met en oeuvre, pour procéder à l'amplification, une ADN polymérase ou une ARN polymérase.

14. Procédé selon l'une quelconque des revendications 1 à 13, **caractérisé en ce que**, dans le cas où l'on procède à l'amplification en employant l'ADN polymérase, on réalise une réaction en chaîne de la polymérase (RCP), et dans le cas où on procède à l'amplification en employant l'ARN polymérase, on réalise une amplification à base de séquences d'acides nucléiques (NASBA) isotherme.

15. Procédé selon l'une quelconque des revendications 1 à 14, **caractérisé en ce que** l'échantillon destiné a l'analyse est constitué de sang, et **en ce que** l'on analyse lors du procédé précité d'une part un échantillon de sang veineux et d'autre part un échantillon de sang artériel, et **en ce que** l'on compare les résultats obtenus.

16. Procédé selon l'une quelconque des revendications 1 à 15, **caractérisé en ce que** l'échantillon destiné à l'analyse est constitué de sang, et **en ce que** l'on analyse lors du procédé précité d'une part un échantillon de sang prélevé dans l'extrémité du doigt et d'autre part un échantillon de sang veineux ou artériel, et **en ce que** l'on compare les résultats obtenus.

17. Procédé selon l'une quelconque des revendications 1 à 16, **caractérisé en ce que** les cellules tumorales proviennent de métastases, de préférence de micrométastases, issues de tumeurs malignes.

18. Procédé selon l'une quelconque des revendications 1 à 17, **caractérisé en ce que** les cellules tumorales proviennent d'un groupe formé par les cellules provenant des tumeurs métastasantes et/ou des néoplasies qui sont elles-mêmes issues d'un lymphocyte T-lymphoblastome, de cellules provenant d'une leucémie lymphocyte T, de cellules de la leucémie myélogène chronique, de cellules de la leucémie lymphatique aiguë, de cellules de la leucémie lymphatique chronique, d'un tétratocarcinome, d'un mélanome, d'un carcinome pulmonaire, d'un cancer colorectal, d'un cancer du sein, d'un carcinome hépato-cellulaire, d'une tumeur du rein, d'une tumeur surrénale, d'un carcinome de la prostate, d'un neuroblastome, d'une tumeur du cerveau, d'un rhabdomyosarcome, d'un léiomyosarcome et/ou d'un lymphome.

19. Amorce oligonucléotide présentant la séquence
5' GACTCGGCTC ACACATGCAG TTCGC 3' (TM1);
5' CTGGTCGAGA TCTACCTTGG GAGAAGC 3' (TM2);
5' ATAAGAATGC GGCCGCGGGT TGCGGAGGGT GGGCCTGGGA GGG 3' (TMK1);
5' CCCAAGCTTG TGGGGGTTAT ATCCTA 3' (TMK2);
5' CGCGGATCCA CTTAGGTCAT CGATCTGCCA ATTTGCAGCA CACT 3' (TMK3);
et/ou
5' CGCGGATCCG ACTTGGTACC ATGAATGGGC AGTGAGCCGG 3' (TMK4).

20. Oligonucléotide présentant la séquence
5' CCATCGATTC CCGTCGCCAA ATCGAGCGGG TACCCC 3' (Kb)
3' GGTAGCTAAG GGCAGCGGTT TAGCTCGCCC ATGGGG 5';
et/ou
5' CCATCGATCG TCCAATCGCT ATACTCTCGG TACCCC 3' (Kc)
3' GGTAGCTAGC AGGTTAGCGA TATGAGAGCC ATGGGG 5'.

21. Acide nucléique étalon pour la co-amplification présentant la séquence selon la figure 7, 8 ou 9 ou son ADNc correspondant.

22. Structure d'acide nucléique pGEM-hTR selon les figures 5a et 5b.

23. Structure d'acide nucléique pGEM-hTR (Ka) selon les figures 6a et 6b.

24. Trousse pour la quantification de cellules tumorales dans un volume de fluide corporel, contenant
(a) pour effectuer la co-amplification, un ou des acides nucléiques étalons, et
(b) pour effectuer l'amplification spécifique de l'acide nucléique qui code pour la télomerase et du ou des acides nucléiques selon (a), un couple d'amorces oligonucléotides,
**caractérisée en ce que** le ou les acides nucléiques étalons selon (a) présentent une séquence selon la figure 7, 8 et/ou 9, et **en ce que** le couple d'amorces oligonucléotides selon (b) présente les séquences suivantes:
5' GACTCGGCTC ACACATGCAG TTCGC 3' (TM1)
et
5' CTGGTCGAGA TCTACCTTGG GAGAAGC 3' (TM2),
où TM1 et/ou TM2 contiennent éventuellement une séquence de promoteur pour une ARN polymérase.

25. Trousse selon la revendication 24, **caractérisée en ce qu'**elle contient de plus un oligonucléotide marqué pour la détection de l'acide nucléique amplifié provenant de l'échantillon destiné à l'analyse et/ou un ou plusieurs oligonucléotides marqués pour la détection du ou des acides nucléiques étalons co-amplifiés, en particulier les oligonucléotides présentant les séquences:
5' CGACTTTGGA GGTGCCTTCA 3' 0(wt)
5' AAGTCGGATC CACTTAGGTC 3' 0(Ka)
5' CGCTCGATTT GGCGACGGGA 3' 0(Kb)
5' GAGAGTATAG CGATTGGACG 3' 0(Kc)
et leurs séquences complémentaires inverses correspondantes.

26. Trousse selon l'une quelconque des revendications 24 à 25, **caractérisée en ce qu'**elle contient de plus une transcriptase inverse, une ADN polymérase, de préférence une Taq polymérase, une ADNase et/ou des tampons appropriés et éventuellement des agents aptes à diminuer la concentration en cellules souche et/ou en cellules immunitaires activées et/ou aptes à augmenter la concentration en cellules tumorales.

27. Trousse selon l'une quelconque des revendications 24 à 26, **caractérisée en ce qu'**elle contient de plus une transcriptase inverse, une ARN polymérase, de préférence une T7 ARN polymérase, une ARNase H, une ADNase et/ou des tampons appropriés et éventuellement des nucléotides marqués ainsi qu'éventuellement des agents aptes à diminuer la concentration en cellules souche et/ou en cellules immunitaires activées et/ou aptes à augmenter la concentration en cellules tumorales.
